(19) Europäisches Patentamt
European Patent Office
Office européen des brevets

(11) Publication number: **0 026 990**
**B1**

(12) # EUROPEAN PATENT SPECIFICATION

(45) Date of publication of patent specification: **01.02.84**

(51) Int. Cl.$^3$: **C 07 D 405/06**, A 01 N 43/50, A 01 N 43/64

(21) Application number: **80303218.4**

(22) Date of filing: **12.09.80**

(54) **Fungicidal dioxolanyl- and dioxanyl-methazolium derivatives, compositions comprising them, process for preparation thereof, and uses thereof.**

(30) Priority: **12.09.79 CH 8254/79**

(43) Date of publication of application:
**15.04.81 Bulletin 81/15**

(45) Publication of the grant of the patent:
**01.02.84 Bulletin 84/5**

(84) Designated Contracting States:
**AT BE CH DE FR GB IT LI NL SE**

(56) References cited:
**GB - A - 2 026 486**
**US - A - 3 575 999**
**US - A - 3 936 470**

**The file contains technical information submitted after the application was filed and not included in this specification**

(73) Proprietor: **JANSSEN PHARMACEUTICA N.V.**
**Turnhoutsebaan 30**
**B-2340 Beerse (BE)**

(72) Inventor: **Kunz, Walter**
**79 Gleneagles Road**
**Flixton Manchester M31 2SA (GB)**
Inventor: **Sturm, Elmar**
**Ziegelbundtenweg 20B**
**CH-4147 Aesch (CH)**
Inventor: **Hubele, Adolf**
**Obere Egg 9**
**CH-4465 Magden (CH)**
Inventor: **Gloor, Bernhard**
**Hohenweg 10**
**CH-4133 Pratteln (CH)**
Inventor: **Heeres, Jan**
**Leemskuilen 18**
**B-2350-Vosselaar (BE)**

(74) Representative: **Jones, Alan John et al,**
**CARPMAELS & RANSFORD 43 Bloomsbury Square**
**London, WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 026 990 B1

# Fungicidal dioxolanyl- and dioxanyl-methazolium derivatives, compositions comprising them, process for preparation thereof, and uses thereof

This invention relates to fungicidal dioxolanyl- and dioxanyl-methazolium derivatives, to compositions comprising them, to a process for the preparation thereof, and to uses thereof.

The present invention provides fungicidal compounds of Formula I

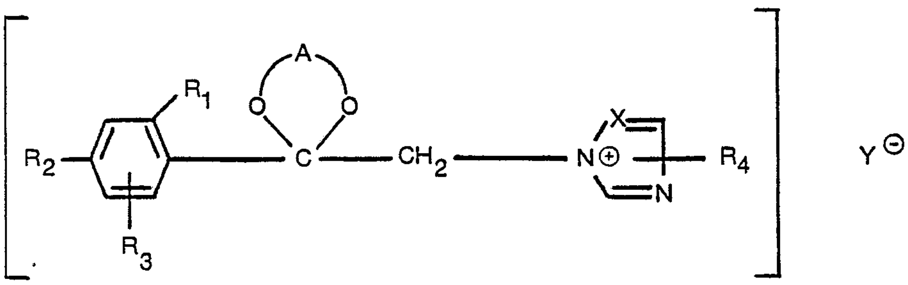

(I)

wherein

$R_1$, $R_2$, and $R_3$ represent independently of one another hydrogen or halogen, at least one residue being a halogen;

A represents one of the following groups (a), (b), (c) and (d):

(a) 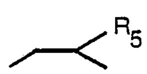 (c) 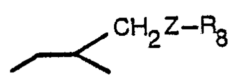

(b) 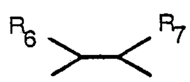 (d) 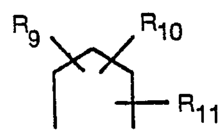

Z represents O or S:

$R_5$ represents hydrogen or $C_1$—$C_6$ alkyl, possibly substituted with a halogen;

$R_6$ represents methyl or ethyl;

$R_7$ represents methyl, ethyl, or propyl, or

$R_6$ and $R_7$ together form a tetramethylene group which can be substituted by 1 or 2 methyl groups;

$R_8$ represents $C_1C_6$ alkyl, $C_3$—$C_4$ alkenyl, 2-propynyl-, 3-halo-2- propynyl, $C_1$—$C_6$ alkoxy-$C_1$—$C_6$-alkyl, or phenyl or benzyl possibly substituted singly, doubly, or triply by halogen, alkyl, alkoxy, nitro, cyano, or trifluoromethyl;

$R_9$ and $R_{10}$ represent independently of one another hydrogen or $C_1$—$C_4$ alkyl and

$R_{11}$ represents hydrogen, $C_1$—$C_4$ alkyl, or —$CH_2OH$, the total number of C-atoms in $R_9$, $R_{10}$, and $R_{11}$ not exceeding 10;

X represents —CH= or —N=;

$R_4$ represents $C_1$—$C_{16}$ alkyl, possibly substituted singly or multiply by halogen, CN, SCN, COO($C_1$—$C_3$)-alkyl, phenyl, or napthyl and/or possibly interrupted by O, S,

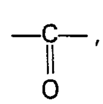

or $SO_2$, $C_3$—$C_6$ alkenylmethyl possibly substituted by a halogen or an alkylthio group, or $C_3$—$C_6$ alkynylmethyl possibly substituted by a halogen, the phenyl or naphthyl group substituent possible in $R_4$ being also possibly substituted singly or multiply by halogen, $NO_2$, CN, OH, $C_1$—$C_4$ alkyl, methoxy, trifluoromethyl, or phenyl; and

Y represents the anion of an organic or inorganic acid.

Where A falls into one of the groups (a), (b) or (c) then the compounds in Formula I stand for quarternary dioxolane. Where A represents the group mentioned in (d), then the compounds stand for quarternary dioxane.

By alkyl or an alkyl part of another substituent are meant, according to the number of carbon atoms specified, for example the following groups: methyl, ethyl, propyl, butyl, pentyl, hexyl, heptyl, octyl, nonyl, decyl, undecyl, dodecyl, hexadecyl, and their isomers such as isopropyl, isobutyl, sec.butyl, tert.butyl, 3-ethylpentyl, and 3-methylbutyl.

The O- or S-atoms and the CO— or $SO_2$ -groups possibly present in residue $R_4$ can occur in the alkyl residue itself, but also between the alkyl residue and a possible substituent.

By alkenyl are meant for example allyl, 2-butenyl, and 3-methyl-2-butenyl, whereas alkynyl represents, inter alia, propargyl.

The halogen stands for fluorine, chlorine, bromine, or iodine.

The anions are derived, for example, from hydrohalic acids, such as hydrochloric, hydrobromic, or hydroiodic acid, fluoroboric acid, nitric acid, phosphoric acid, thiophosphoric or dithiophosphoric acid, sulfuric acid, methanesulfonic acid, ethanesulfonic acid, acetic acid, haloacetic acids, propionic acid, halopropionic acids, butyric acid, oxalic acid, picric acid, p-toluenesulfonic acid, or benzensulfonic acid.

The triazole compounds of Formula I (X=N) are clearly preferred as fungicides.

The compounds of Formula I can be prepared by reacting a compound of Formula II

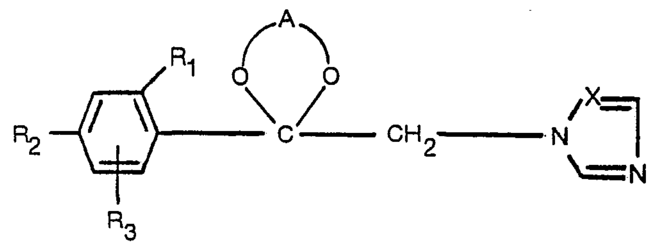

(II)

with a compound of Formula III

$$Y—R_4 \qquad \text{(III)}$$

where $R_1$, $R_2$, $R_3$, $R_4$, A, X, and Y have the same meanings as in Formula I.

The reactions can be performed in the presence or absence of protic or aprotic solvents inert to the reactants. For example, the following can be used: alcohols such as ethanol; nitriles such as acetonitrile; ethers such as dioxane, dimethoxyethane, or t-butyl methyl ether; halohydrocarbons such as chloroform, trichlorethane, etc. The reactions are performed at normal pressure or at a slightly elevated pressure and at temperatures between 0 and 180°C and preferably between 50 and 120°C.

This method also constitutes a part of the invention.

In a further variant of the process, and if so desired, the anion Y in an already obtained compound of Formula I in accordance with basically known methods can be exchanged for another anion, for example by converting a quarternary azalium halide of Formula I into the corresponding azalium hydroxide with a base or an anion exchanger and reacting it with an acid (cf. Houben-Weyl 11/2, 620).

The starting compounds of Formula II are known in particular from U.S. Patents 3,575,999; 3,936,470; 4,101,666; 4,106,664; 4,156,008, Swiss Applications 7963/78; 8001/78; 8005/78; 8041/78, and U.S. Application 53,640, and analogs can be prepared according to the methods described therein. The compounds of Formula I have a better action when compared with these starting substances already familiar as fungicides. Thus, undesirable plant-regulatory excess effects and the phytotoxicity sometimes caused by the compounds of Formula II can be largely eliminated.

The Formula I compounds can be used on their own or together with suitable carriers and/or admixtures that can be solid or liquid and correspond to the substances usual in formulation techniques such as natural or regenerated minerals, solvents, dispersants, wetting agents, adhesion agents, thickeners, binding agents, or fertilizers. Substances of this kind are manufactured in a basically known manner by intimate mixing and grinding of the components. The Formula I compounds are found in the following practical forms (the data on the weight percentages representing appropriate quantities of the active ingredients).

Solid forms

dusting or spraying materials, (up to 10%) granulates, coated granulates, impregnation granulates, and homogeneous granulates; pellets (grains) (1 to 80%)

Liquid forms:

(a) dispersable in water

active ingredient concentrates:

wettable powders, pastes, 25—90%, in the commercial pack, 0.01 to 15% in ready-for-use solution, emulsions; solution concentrates (10 to 50%; 0.01 to 15% in ready-for-use solution)

(b) Solutions: aerosols.

The content of active ingredient in commercial products lies between 0.1 to 95 percent (w/w), the content of additives from 5 to 99.9 percent (w/w), whereby, in general, 0 to 30 percent (w/w) tenside can be found included in the additives.

By tensides should hereby be understood contact surface- or surface- active agents which are

mostly dissolved or dispersed in a liquid and are adsorbed particularly on contact surfaces. A tenside molecule contains at least one group which shows affinity to substances of stronger polarity — which in general causes the solubility in water — and at least one other group with less affinity to water. Tensides are therefore molecules with one lipophile = hydrophobe, i.e. molecule part which lacks affinity to water or has an affinity to lipids, mostly one carboxyl part with alkyl- and/or aryl-components and furthermore with one hydrophile = lipophobe, i.e. molecular part which has an affinity to water and lacks affinity to lipids, e.g. a perfluoralkyl part. Generally, products used in practical work are mostly mixtures of such compounds. Tensides not only make possible a good distribution of the active substance in a liquid, e.g. aqueous medium, but also an increased wettability of the plants. This leads to a reduction of the active ingredient in the finished product and thereby to a smaller environmental burden.

The Formula I compounds can, of course, be used with other suitable pesticides to broaden the action spectrum, e.g. with fungicides, bactericides, insecticides, acaricides, herbicides, or agents that influence plant growth.

The discovery covers also substances which contain at least one active substance as a component in Formula I, as well as the application of the substance to the fight against — and/or prevention of — an attack of microorganism. Furthermore, the present invention, also includes the manufacturing of the substance, which can be recognized through the thorough mixing of the active substance with one or several substances described herein, respectively function groups. Included is also a procedure for the fight against microorganisms, which distinguishes itself through the application of the compounds of Formula I, resp. the new substance.

Surprisingly, it has been found that compounds with the structure of Formula I exhibit a microbicide spectrum for the protection of cultivated plants that is very beneficial for practical requirements. The cultivated plants within the scope of the present invention may be, for example:

Cereal crops: (wheat, barley, rye, oats, rice); Beets: (sugar and fodder beets); pip fruits, stone fruits, and berry fruits: (apples, pears, plums, peaches, almonds, cherries, strawberries, raspberries, and blackberries); legumes: (beans, lentils, peas, soya); oil cultures: (rape, mustard, poppyseed, olives, sunflowers, coconut trees, castor-oil plants, cocoa trees, peanuts); cucumber plants: (pumpkins, cucumbers, melons); fibrous plants (cotton, flax, hemp, jute), citrus fruits: (oranges, lemons, grapefruits, tangerines); types of vegetables: (spinach, head lettuces, asparagus, types of cabbage, carrots, onions, tomatoes, potatoes, red peppers) or plants such as maize, tobacco, nuts, coffee, sugar cane, grapevines, hops, banana, natural rubber, and ornamental plants.

The use of the active ingredients of Formula I on plants or parts (fruit, blossoms, foliage, stems, tubers, roots) of these and related useful cultures can check or destroy the occurrence of fungi, parts of the plant growing subsequently also being protected from such fungi. The active principles are effective against the phytopathogenic fungi of the following classes: ascomycetes (e.g. Erysiphaceae, Podosphaera, Venturia; basidomycetes, in particular rust fungi (e.g. Puccinia); fungi imperfecti (e.g. Botyrtis and Moniliales inter alia Cercospora), and the Omycetes of the class of Phycomycetes. In addition, the Formula I compounds sometimes have a systemic action. They can also be used as disinfectants for the treatment of seed (fruit, tubers, grains) and plant cuttings for protection against fungal infections and against phytopathogenic fungi occurring in the soil. The invention thus also comprises the use of the Formula I compounds to combat phytopathogenic microorganisms.

The following substituents and combinations of these are preferred:

with $R_1$, $R_2$, $R_3$ 2,4-dichloro-(the phenyl group is bound to the rest of the molecule in the 1-position) with

a) $R_5$ = hydrogen or $C_1$—$C_4$ alkyl

b) $R_6$ and $R_7$ methyl or (together) tetramethylene

c) Z = 0, $R_8$ = $C_1$—$C_3$ alkyl or phenyl with $R_4$ (i)

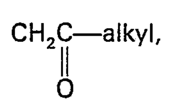

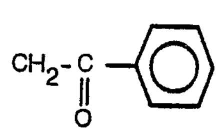 (possibly substituted)

(ii) $CH_2$—CH=$CH_2$, 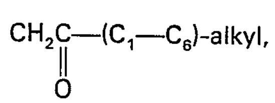

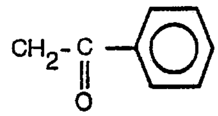 (possibly substituted by Cl, $NO_2$, CN, OH, $CH_3$, $OCH_3$, $CF_3$);

with X —N=
with Y a halide, sulfate, nitrate, phosphate, methanesulfonate, ethanesulfonate, p-toluenesulfonate, or benzenesulfonate anion.

Thus, it is found that the following groups are preferred:
$R_1 = R_2 =$ chlorine
$R_3 =$ hydrogen

A = a) 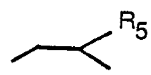

b) 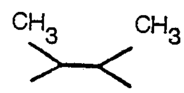 or 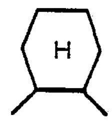

c) 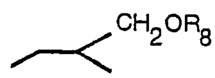

d) 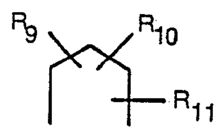

where
$R_5$ is hydrogen or $C_1$—$C_4$ alkyl
$R_8$ is $C_1$—$C_3$ alkyl or phenyl
$R_9$ and $R_{10}$ are independently of one another hydrogen or $C_1$—$C_4$ alkyl, and
$R_{11}$ is hydrogen, $C_1$—$C_4$ alkyl, or $CH_2OH$, the total number of carbon atoms in $R_9$, $R_{10}$, and $R_{11}$ not exceeding ten,
X represents —N=,
$R_4$ is $C_3$—$C_6$-alkenylmethyl, $C_3$—$C_6$-alkynylmethyl,

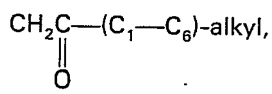

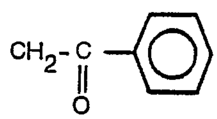

(possibly substituted by Cl, $NO_2$, CN, OH, $CH_3$, $OCH_3$), or $CH_2CN$ and

Y represents a halide, sulfate, nitrate, phosphate, methanesulfonate, ethanesulfonate, p-toluenesulfonate, or benzenesulfonate anion.

Furthermore, the compounds of the following subgroup Ib are favoured:
$R_1 =$ hydrogen or chlorine
$R_2 =$ chlorine
$R_3 =$ hydrogen

A = e) 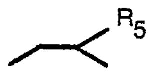

f) 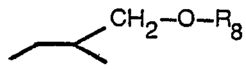

g) 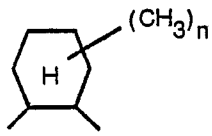 n = 0 or 1

h) 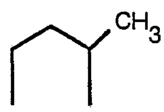

i) 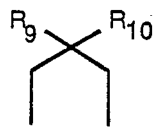

wherein $R_5$ means ethyl or n-propyl, $R_8$ represents $C_1$—$C_3$ alkyl or phenyl, $R_9$ means hydrogen or methyl, and $R_{10}$ means hydrogen or $C_1$—$C_3$ alkyl,

$X = N$

$R_4$ = —$CH_2$ —CO—$R_{12}$ or —CH($COOR_{13}$),

wherein $R_{12}$ is $C_1$—$C_4$ alkyl or one unsubstituted phenyl-radical or one phenyl-radical substituted by alkyl, halogen, $NO_2$, CN, OH, $OCH_3$, $CF_3$ singly or multiply;
wherein $R_{12}$ further means —$CH_2$—CO— $R_{13}$, —$CH_2$—$COOR_{13}$ or —$OR_{14}$; $R_{13}$ stands for $C_1$—$C_4$ alkyl; and $R_{14}$ means alkyl, alkenyl or alkynyl each with maximum of 6 C-atoms or an unsubstituted phenyl radical or a substituted one by $C_1$—$C_4$ alkyl and/or halogen.

In the structure element $R_4$ = —$CH_2$—CO—$R_{12}$ either the hydrogen can be substituted by lower alkyl or the CO-group can be substituted by $SO_2$. In principle such groups $R_4$ will behave comparably to the groups $R_4$ in the subgroup Ib.

The activity progression of a compound of Formula I in relation to a compound of Formula II cannot be derived from the molecular structure, because compounds $R_4Y$ of Formula III do not contain this fungicide activity. One equimolar mixture of a compound of Formula II and one compound of Formula III exhibits, as expected, a weaker fungicide activity than a compound of Formula II by itself.

It is therefore surprising that the quarternarization of compounds of Formula II with fungicidally inactive compounds leads $R_4Y$ to fungicides with favourable spectrum and decreasing phytotoxic side effects.

Favoured active substances are, e.g. the below listed compounds no. 1.9, 1.10, 1.15, 1.18, 1.20, 1.47, 1.48, 1.49, 1.57, 1.80, 1.81, 1.84, 1.85, 1.86, 1.88, 2.10, 2.11, 2.12, 4.7, 4.23.

These preferred groups are obviously part of the invention as well.

The following Examples serve the purpose of explaining the invention without limiting its scope. The data on temperature are in degrees centigrade, the data on pressure in millibars, and the data on parts and percentages refer to weight.

Preparation Example

Example 1

Process for the manufacture of the quaternary salts of 1-[2-(2,4-dichlorophenyl)-4-n-propyl-1,3-dioxolan-2-ylmethyl]-1H-1,2,4-triazole of formula

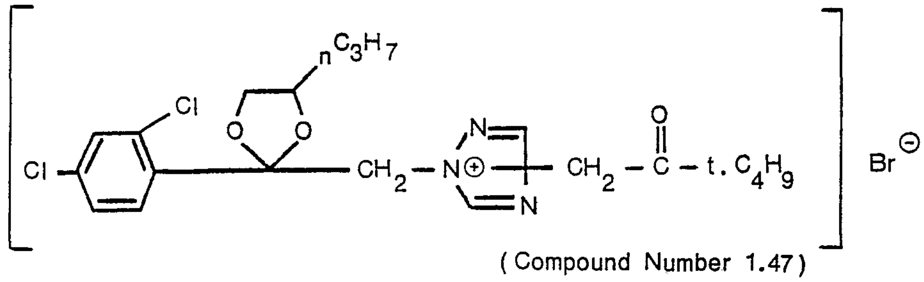

(Compound Number 1.47)

19 g of the compound 1-[2-(2,4-dichlorophenyl)-4-n-propyl-1,3-dioxolan-2-yl-methyl]-1H-1,2,4-triazole and 9 g of bromomethyl tert.butyl ketone were boiled in 700 ml of ethanol overnight under reflux. The mixture was then boiled down, the resin left behind was digested with ether, filtered under suction, and washed well with ether. The white precipitate melted at 156—158°C.

The following Formula I compounds can be prepared in a similar fashion by using one of the methods described herein.

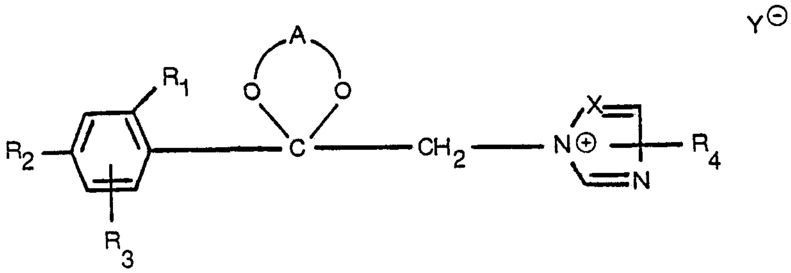

TABLE 1

($R_1$ = $R_2$ = chlorine, $R_3$ = H; A = 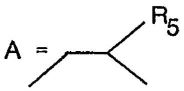

| Compound No. | $R_5$ | X | $R_4$ | Y | Phys. data |
|---|---|---|---|---|---|
| 1.1 | H | N | $-CH_3$ | I | M.p. 196° |
| 1.2 | H | N | 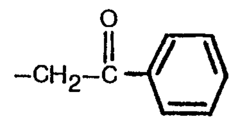 | Br | M.p. 175.3° |
| 1.3 | H | N | 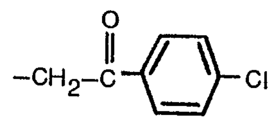 | Br | M.p. 187° |
| 1.4 | H | N | 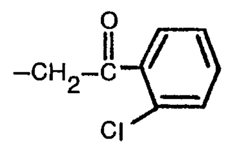 | Br | M.p. 205.9° |
| 1.5 | H | N | 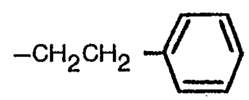 | Br | M.p. 241.1° |

TABLE 1 (Continued)

| Compound No. | $R_5$ | X | $R_4$ | Y | Phys. data |
|---|---|---|---|---|---|
| 1.6 | H | N | 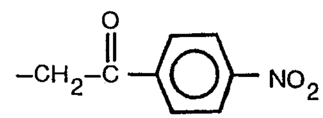 | Br | M.p. 227–8° |
| 1.7 | $C_3H_7$-n | N | 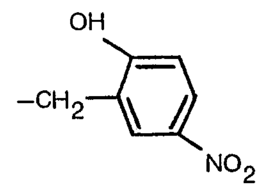 | Cl | M.p. 214–9° |
| 1.8 | $C_2H_5$ | N | $-CH_2-C\equiv CH$ | Br | M.p. 145–8° |
| 1.9 | $C_2H_5$ | N | 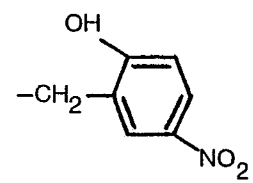 | Cl | M.p. 213–7° |
| 1.10 | $C_3H_7$-n | N | 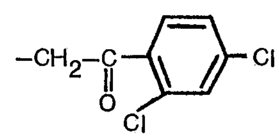 | Br | M.p. 152–160° |

TABLE 1 (Continued)

| Compound No. | $R_5$ | X | $R_4$ | Y | Phys. data |
|---|---|---|---|---|---|
| 1.11 | $C_2H_5$ | CH | 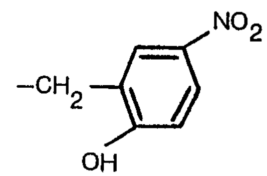 | Cl | M.p. 99–115° |
| 1.12 | $C_2H_5$ | N | 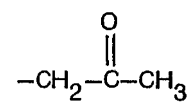 | Cl | M.p. 194–5° |
| 1.13 | $C_2H_5$ | CH | $-CH_3$ | I | M.p. 168–70° |
| 1.14 | $C_2H_5$ | CH | 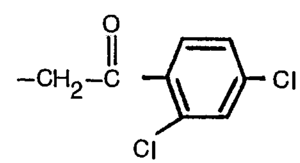 | Br | M.p. 165–9° |
| 1.15 | $C_2H_5$ | N | 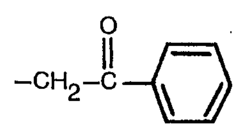 | Br | M.p. 136–9° (Dec.) |

TABLE 1 (Continued)

| Compound No. | $R_5$ | X | $R_4$ | Y | Phys. data |
|---|---|---|---|---|---|
| 1.16 | $C_2H_5$ | N | 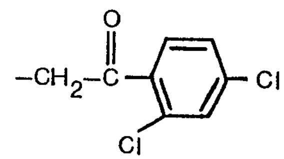 | Br | M.p. 192–4° |
| 1.17 | $C_2H_5$ | N | $-CH_2CH=CH_2$ | Br | M.p. 130–4° |
| 1.18 | $C_3H_7$-n | N | 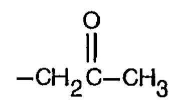 | Cl | M.p. 145–9° |
| 1.19 | $C_2H_5$ | CH | $-CH_2-C\equiv CH$ | Br | M.p. 182–190° |
| 1.20 | $C_3H_7$-n | N | 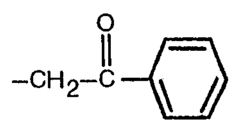 | Br | M.p. 90–120° |
| 1.21 | $C_2H_5$ | CH | $-CH_2-CH=CH_2$ | Br | M.p. 170–195° |
| 1.22 | $C_3H_7$-n | N | $-CH_2-CH=CH_2$ | Br | M.p. 132–8° |
| 1.23 | $C_3H_7$-n | N | $-CH_2-C\equiv CH$ | Br | M.p. 151–4° |

TABLE 1 (Continued)

| Compound No. | $R_5$ | X | $R_4$ | Y | Phys. data |
|---|---|---|---|---|---|
| 1.24 | $C_2H_5$ | N | 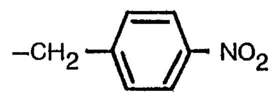 | Br | M.p. 217–20° |
| 1.25 | $C_3H_7$-n | N | 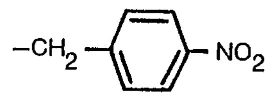 | Br | M.p. 213–5° |
| 1.26 | $C_2H_5$ | CH | 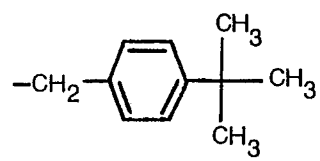 | Br | M.p. 140–146° |
| 1.27 | $C_2H_5$ | CH | 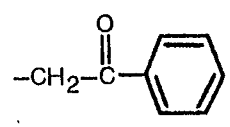 | Br | solid |
| 1.28 | $C_2H_5$ | CH | $-C_2H_5$ | I | M.p. 150–1° |
| 1.29 | $CH_3$ | N | 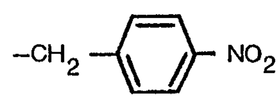 | Br | |

TABLE 1 (Continued)

| Compound No. | $R_5$ | X | $R_4$ | Y | Phys. data |
|---|---|---|---|---|---|
| 1.30 | $C_2H_5$ | CH | 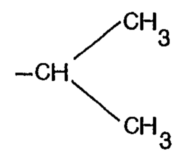 | I | M.p. 200–203° |
| 1.31 | $C_2H_5$ | CH | 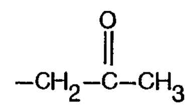 | Cl | oil |
| 1.32 | $C_2H_5$ | CH | $-CH_2(CH_2)_{10}CH_3$ | Br | M.p. 105–108° |
| 1.33 | $C_3H_7$-n | N | $-C_2H_5$ | I | M.p. 146–8° |
| 1.34 | $C_2H_5$ | CH | 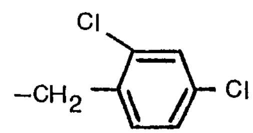 | Cl | solid |
| 1.35 | $C_3H_7$-n | N | $-CH_3$ | I | M.p. 143–6° |
| 1.36 | $C_3H_7$-n | N | 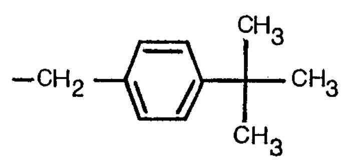 | Br | M.p. 155–6° |

TABLE 1 (Continued)

| Compound No. | $R_5$ | X | $R_4$ | Y | Phys. data |
|---|---|---|---|---|---|
| 1.37 | $C_2H_5$ | N | $-C_2H_5$ | I | M.p. 120–3° |
| 1.38 | $C_2H_5$ | N | $-CH_3$ | I | M.p. 143–5° |
| 1.39 | $C_2H_5$ | N | 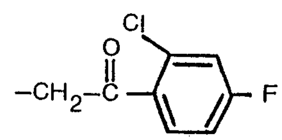 | Br | M.p. 150–152° |
| 1.40 | $C_3H_7$-n | CH | $-CH_3$ | I | M.p. 154–161° |
| 1.41 | $C_3H_7$-n | CH | 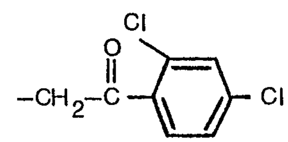 | Br | M.p. 156–160° |
| 1.42 | $C_3H_7$-n | CH | $-CH_2-C\equiv CH$ | Br | M.p. 170–4° |
| 1.43 | $C_3H_7$-n | CH | 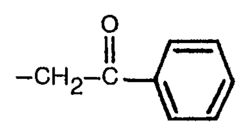 | Br | M.p. 59–70° |

TABLE 1 (Continued)

| Compound No. | $R_5$ | X | $R_4$ | Y | Phys. data |
|---|---|---|---|---|---|
| 1.44 | $C_3H_7$-n | CH | $-CH_2-CH=CH_2$ | Br | M.p. 109–121° |
| 1.45 | $C_2H_5$ | N | 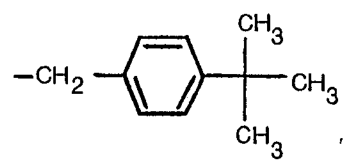 | Br | M.p. 87–100° |
| 1.46 | $C_2H_5$ | N | 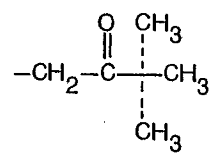 | Br | M.p. 178° |
| 1.47 | $C_3H_7$-n | N | 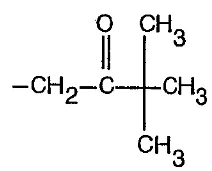 | Br | M.P. 156–8° |
| 1.48 | $C_2H_5$ | N | 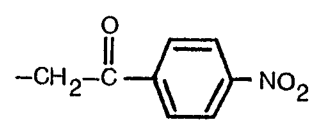 | Br | M.p. 148–150° |

TABLE 1 (Continued)

| Compound No. | $R_5$ | X | $R_4$ | Y | Phys. data |
|---|---|---|---|---|---|
| 1.49 | $C_3H_7$-n | N | 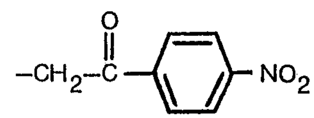 | Br | M.p. 143–7° |
| 1.50 | $C_2H_5$ | N | 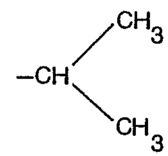 | I | oil |
| 1.51 | $C_2H_5$ | N | $-CH_2CN$ | Cl | M.p. 197–200° |
| 1.52 | $C_3H_7$-n | CH | 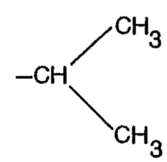 | I | M.p. 152–161° |
| 1.53 | $C_3H_7$-n | CH | $-CH_2CN$ | Cl | M.p. 197–200° |
| 1.54 | $C_3H_7$-n | CH | 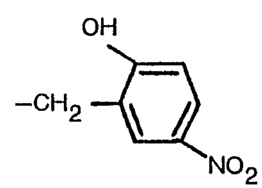 | Cl | M.p. 105–130° |

TABLE 1 (Continued)

| Compound No. | $R_5$ | X | $R_4$ | Y | Phys. data |
|---|---|---|---|---|---|
| 1.55 | $C_3H_7$-n | CH | $-(CH_2)_{11}-CH_3$ | Br | solid |
| 1.56 | $C_3H_7$-n | CH | $-CH(COOC_2H_5)-(CH_2)_5-CH_3$ | Br | oil |
| 1.57 | $C_2H_5$ | N | 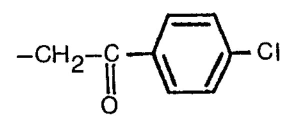 | Br | M.p. 170–2° |
| 1.58 | $C_2H_5$ | N | 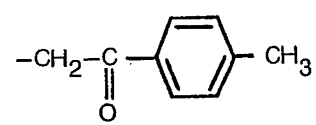 | Br | M.p. 150–5° |
| 1.59 | $C_3H_7$-n | N | 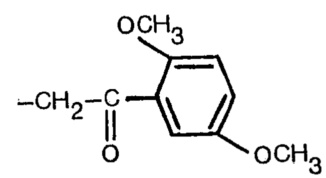 | Br | M.p. 95–130° |

TABLE 1 (Continued)

| Compound No. | $R_5$ | X | $R_4$ | Y | Phys. data |
|---|---|---|---|---|---|
| 1.60 | $C_2H_5$ | N | 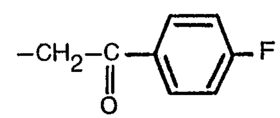 | Cl | M.p. 91–94° |
| 1.61 | $C_3H_7$-n | N | $-CH_2SCN$ | Cl | |
| 1.62 | $C_2H_5$ | N | 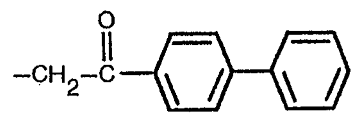 | Br | |
| 1.63 | $C_2H_5$ | N | $-CH_2SO_2CH_3$ | Br | viscous |
| 1.64 | $C_3H_7$-n | N | 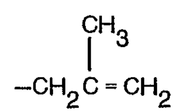 | Cl | |
| 1.65 | $C_2H_5$ | CH | 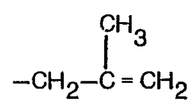 | Cl | |

TABLE 1 (Continued)

| Compound No. | $R_5$ | X | $R_4$ | Y | Phys. data |
|---|---|---|---|---|---|
| 1.66 | $C_3H_7$-n | N | 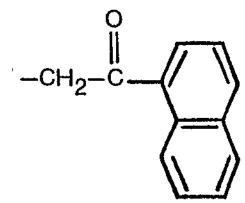 | Br | M.p. 178–180° |
| 1.67 | $C_2H_5$ | N | 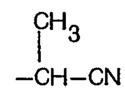 | Br | |
| 1.68 | $C_2H_5$ | N | 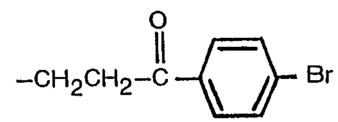 | Cl | resin |
| 1.69 | $C_2H_5$ | N | 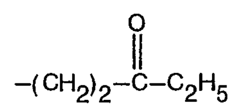 | Cl | |
| 1.70 | $C_3H_7$-n | N | $-CH_2CH_2I$ | I | |
| 1.71 | $C_2H_5$ | N | 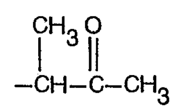 | Br | |

TABLE 1 (Continued)

| Compound No. | $R_5$ | X | $R_4$ | Y | Phys. data |
|---|---|---|---|---|---|
| 1.72 | $C_2H_5$ | N | $-CH_2CH_2CN$ | Br | |
| 1.73 | $C_3H_7$-n | N | $-CH_2CH_2CH_2CN$ | Br | |
| 1.74 | $C_3H_7$-n | N | $-CH_2CH_2SO_2CH_3$ | Cl | resin |
| 1.75 | $C_2H_5$ | N | $-CH_2CH_2OCH=CH_2$ | Cl | |
| 1.76 | $CH_3$ | CH | $-CH_2CH_2CN$ | Cl | |
| 1.77 | $C_4H_9$-n | N | $-CH_2-\overset{O}{\overset{\|}{C}}-CH_3$ | Br | M.p. 161–163° |
| 1.78 | $C_4H_9$-n | CH | 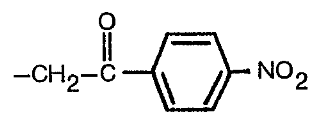 | Cl | |
| 1.79 | $CH_3$ | N | $-CH_2C\equiv CH$ | Cl | |

TABLE 1 (Continued)

| Compound No. | $R_5$ | X | $R_4$ | Y | Phys. data |
|---|---|---|---|---|---|
| 1.80 | $C_2H_5$ | N | 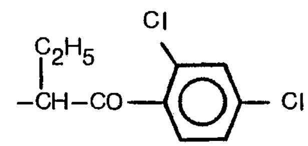 | Br | M.p. 211–213° |
| 1.81 | $C_3H_7$ | N | 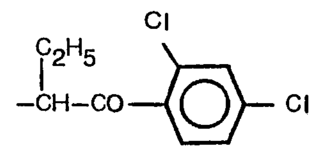 | Br | M.p. 218–221° |
| 1.82 | $C_2H_5$ | N | $-CH(C_6H_5)_2$ | Br | resin |
| 1.83 | $C_2H_5$ | N | 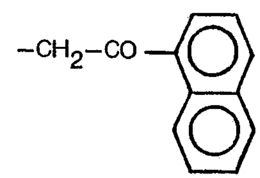 | Br | M.p. 128–132° |
| 1.84 | $C_3H_7$-n | N | 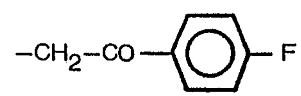 | Cl | M.p. 126–129° |

TABLE 1 (Continued)

| Compound No. | $R_5$ | X | $R_4$ | Y | Phys. data |
|---|---|---|---|---|---|
| 1.85 | $C_3H_7$-n | N | $-CH_2-CH-$ 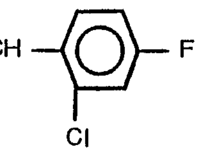 | Br | M.p. 150–155° |
| 1.86 | $C_3H_7$-n | N | $-CH_2-CO-CH_2COOC_2H_5$ | Cl | viscous |
| 1.87 | $C_2H_5$ | N | 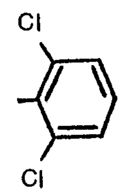 | Br | M.P. 198–200° |
| 1.88 | $C_2H_5$ | N | $-CH_2-CO-CH_2COOC_2H_5$ | Cl | solid |
| 1.89 | $C_3H_7$-i | N | $-CH_2-CO-$ 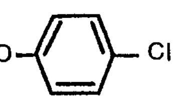 | Br | M.p. 184–188° |

TABLE 1 (Continued)

| Compound No. | $R_5$ | X | $R_4$ | Y | Phys. data |
|---|---|---|---|---|---|
| 1.90 | $C_4H_9$-tert. | N | 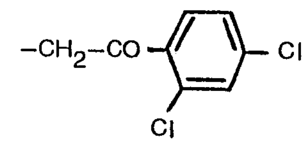 | Br | M.p. 190–192° |
| 1.91 | $C_4H_9$-tert. | CH | 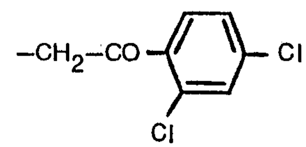 | Br | M.p. 115–120° |
| 1.92 | $C_3H_7$-n | N | $-CH_2-COOC_2H_5$ | Br | resin |
| 1.93 | $C_3H_7$-n | N | $-CH(CH_3)-COOCH_3$ | Br | viscous |
| 1.94 | $C_3H_7$-n | N | $-CH(COOC_2H_5)_2$ | Br | viscous |
| 1.95 | $C_3H_7$-n | N | $-C_4H_9$-n | Br | resin |
| 1.96 | $C_3H_7$-n | N | $-CH_2-COOC_2H_5$ | Cl | viscous |

TABLE 1 (Continued)

| Compound No. | $R_5$ | X | $R_4$ | Y | Phys. data |
|---|---|---|---|---|---|
| 1.97 | $C_2H_5$ | N | 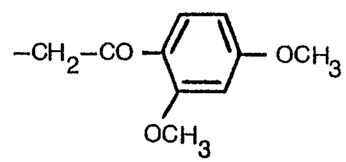 | Br | M.p. 95—103° |
| 1.98 | $C_3H_7$-i | N | $-CH(CH_3)-COOCH_3$ | Br | |
| 1.99 | $C_2H_5$ | N | $-CH_2COOC_2H_5$ | Br | |
| 1.100 | $C_3H_7$-n | N | $-CH(COCH_3)-COOC_2H_5$ | Cl | |
| 1.101 | $C_2H_5$ | N | $-CH_2COOC_4H_9$ | Br | |
| 1.102 | $C_2H_5$ | N | $-CH(CH_3)-COOCH_3$ | Br | |
| 1.103 | $C_2H_5$ | N | $-CH(COCH_3)COOC_2H_5$ | Cl | |

TABLE 2

($R_1 = R_2$ chlorine, $R_3 = H$)   A: 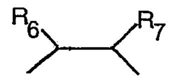

| Compound No. | 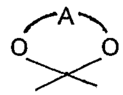 | X | $R_4$ | Y | Phys. data |
|---|---|---|---|---|---|
| 2.1 | 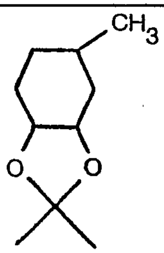 (cis/trans) | N | $-CH_2-COOCH_3$ | Br | viscous |
| 2.2 | 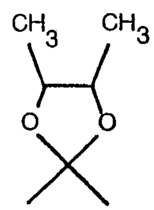 | N | 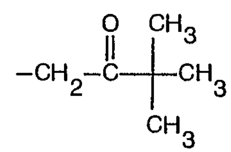 | Br | |
| 2.3 | 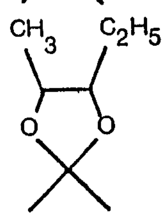 | N | $-CH_2-CH=CH_2$ | Cl | |
| 2.4 | 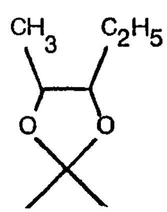 | N | 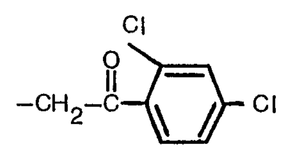 | Cl | |

TABLE 2 (Continued)

| Compound No. | 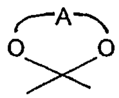 | X | $R_4$ | Y | Phys. data |
|---|---|---|---|---|---|
| 2.5 | 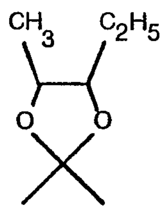 | N | 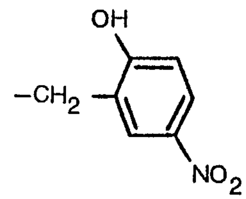 | Br | |
| 2.7 | 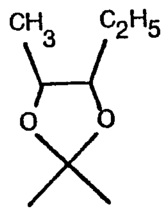 | CH | $-CH_2-CH=CH_2$ | Cl | |
| 2.8 | 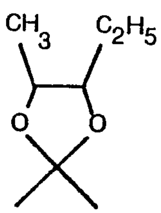 | N | $-CH_2CH_2-SO_2CH_3$ | Cl | resin |
| 2.9 | 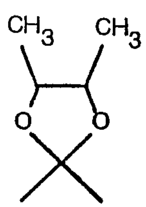 | | 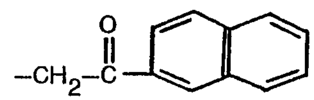 | Br | |

TABLE 2 (Continued)

| Compound No. | 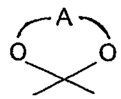 | X | $R_4$ | Y | Phys. data |
|---|---|---|---|---|---|
| 2.10 | 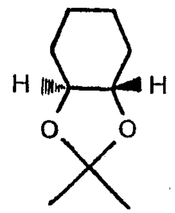 (trans) | N | 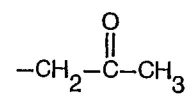 | Br | amorphous |
| 2.11 | 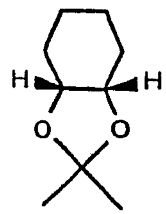 (cis) | N | 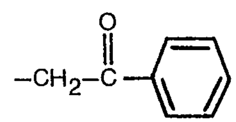 | Br | M.p. 176–178° |
| 2.12 | 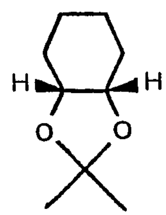 (cis) | N | 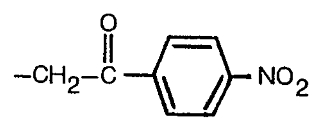 | Br | M.p. 218–220° |

TABLE 3

($R_1 = R_2 =$ chlorine, $R_3 = H$, $Z = O$; A = 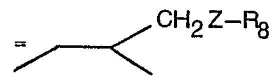 )

| Compound No. | $R_8$ | X | $R_4$ | Y | Phys. data |
|---|---|---|---|---|---|
| 3.1 | $CH_3$ | N | 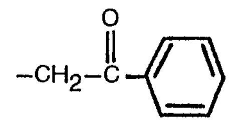 | Br | viscous |
| 3.2 | $(CH_2)_2OCH_3$ | N | 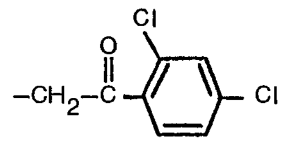 | Cl | viscous |
| 3.3 | 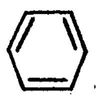 | CH | $-CH_2CN$ | Cl | |
| 3.4 | 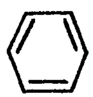 | N | $-CH_2CN$ | Cl | |
| 3.5 | 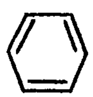 | N | 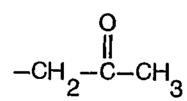 | Cl | viscous |

TABLE 3 (Continued)

| Compound No. | $R_8$ | X | $R_4$ | Y | Phys. data |
|---|---|---|---|---|---|
| 3.6 | 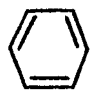 | N | $-CH_2-CH=CH_2$ | Cl | |
| 3.7 | $C_2H_5$ | N | $CH_2COCH_3$ | Br | viscous |

TABLE 4

($R_1$ = $R_2$ = chlorine, $R_3$ = H; A = 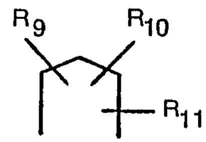)

| Compound No. | 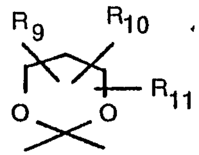 | X | $R_4$ | Y | Phys. data |
|---|---|---|---|---|---|
| 4.1 | 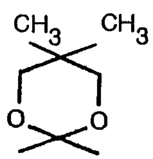 | CH | 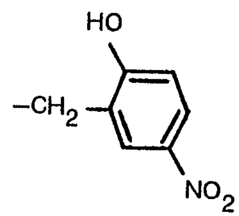 | Cl | 152–68° |
| 4.2 | 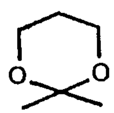 | N | $-CH_3$ | I | |
| 4.3 | 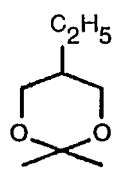 | N | $-CH_2-CH=CH_2$ | Br | |

TABLE 4 (Continued)

| Compound No. | 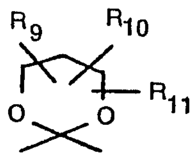 | X | $R_4$ | Y | Phys. data |
|---|---|---|---|---|---|
| 4.4 | 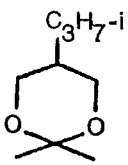 | N | 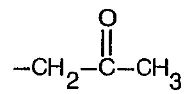 | Cl | |
| 4.5 | 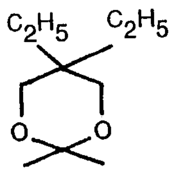 | CH | 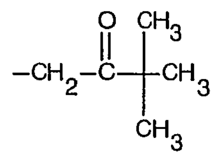 | Cl | |
| 4.6 | 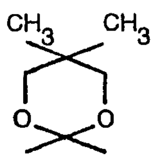 | N | $-CH_2C\equiv CH$ | Br | M.p. 138–142° |
| 4.7 | 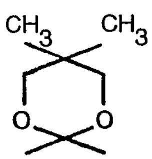 | N | $-CH_2-CH=CH_2$ | Br | amorphous |

TABLE 4 (Continued)

| Compound No. | 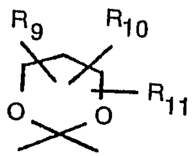 | X | $R_4$ | Y | Phys. data |
|---|---|---|---|---|---|
| 4.8 | 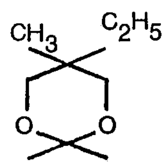 | CH | 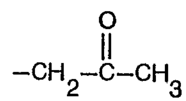 | Cl | |
| 4.9 | 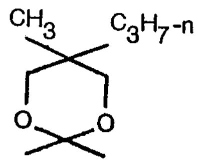 | CH | 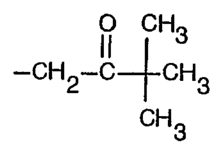 | Br | viscous |
| 4.10 | 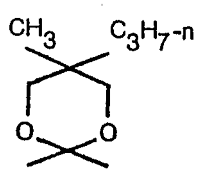 | N | $-CH_2-CN$ | Cl | |
| 4.11 | 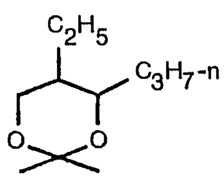 | N | 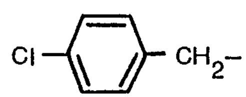 | Cl | |

TABLE 4 (Continued)

| Compound No. | 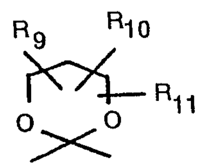 | X | $R_4$ | Y | Phys. data |
|---|---|---|---|---|---|
| 4.12 | 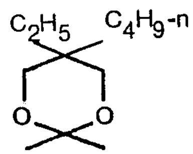 | CH | 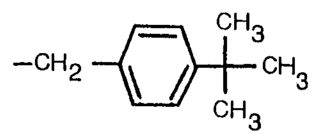 | Br | |
| 4.13 | 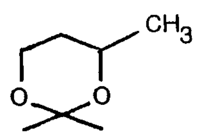 | N | 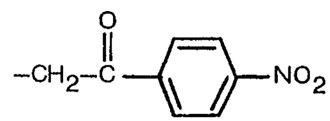 | Br | M.p. 111–115° |
| 4.14 | 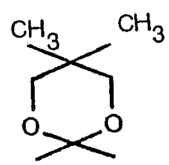 | N | 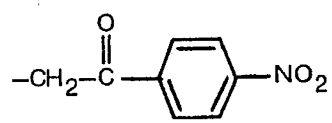 | Br | M.p. 139–142° |
| 4.15 | 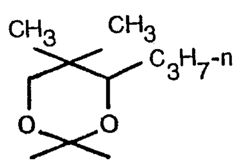 | CH | 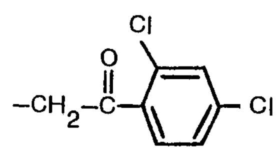 | Br | resin |

TABLE 4 (Continued)

| Compound No. | 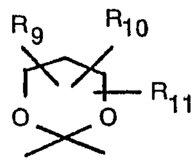 | X | $R_4$ | Y | Phys. data |
|---|---|---|---|---|---|
| 4.16 | 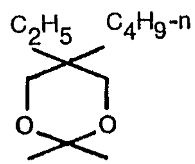 | N | 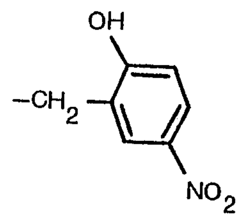 | Cl | |
| 4.17 | 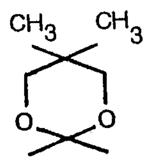 | N | 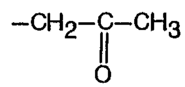 | Cl | |
| 4.18 | 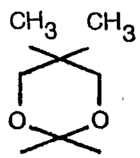 | CH | 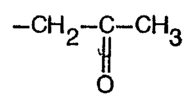 | Br | |
| 4.19 | 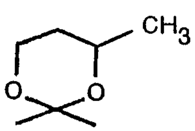 | CH | 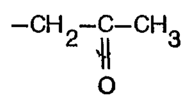 | Br | |

TABLE 4 (Continued)

| Compound No. | 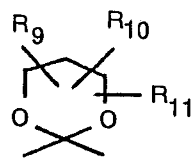 | X | $R_4$ | Y | Phys. data |
|---|---|---|---|---|---|
| 4.20 | 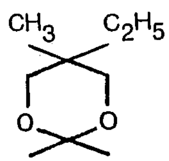 | N | 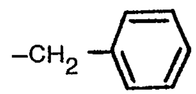 | Cl | |
| 4.21 | 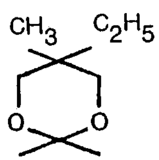 | CH | $-CH_2-CN$ | Cl | |
| 4.22 | 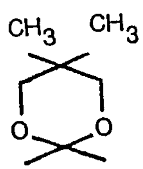 | N | $-CH_2-CN$ | Cl | M.p. 203–6° |
| 4.23 | 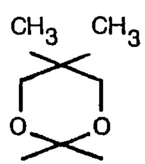 | N | 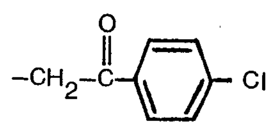 | Br | M.p. 149–152° |

TABLE 4 (Continued)

| Compound No. | 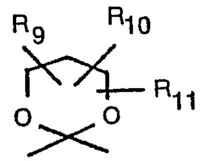 | X | $R_4$ | Y | Phys. data |
|---|---|---|---|---|---|
| 4.24 | 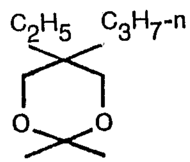 | N | 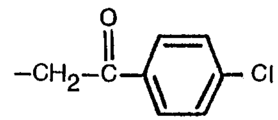 | Cl | |
| 4.25 | 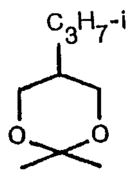 | CH | 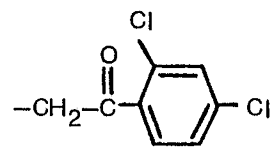 | Br | |
| 4.26 | 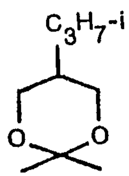 | N | 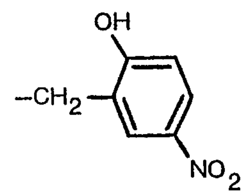 | Cl | |
| 4.27 | 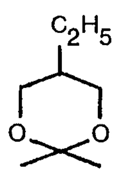 | N | 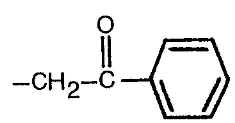 | Br | resin |

TABLE 4 (Continued)

| Compound No. | 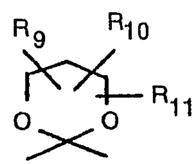 | X | $R_4$ | Y | Phys. data |
|---|---|---|---|---|---|
| 4.28 | 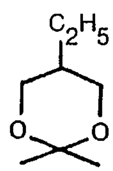 | N | $-CH_2-CN$ | Cl | |
| 4.29 | 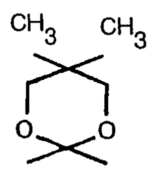 | CH | $-C_4H_9$-n | Br | |
| 4.30 | 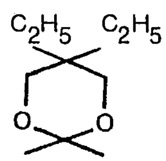 | N | $-C_3H_7$-i | I | |
| 4.31 | 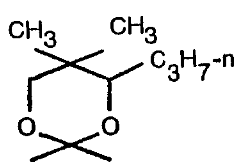 | CH | $-CH_2-CH=CH_2$ | Br | |

TABLE 4 (Continued)

| Compound No. | 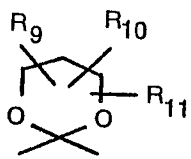 | X | $R_4$ | Y | Phys. data |
|---|---|---|---|---|---|
| 4.32 | 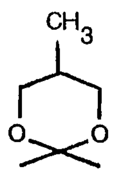 | CH | 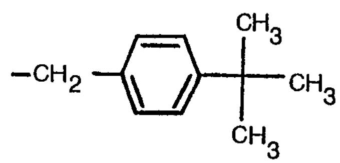 | Br | |

Formulation Examples

*Dusting agents:*

The following materials are used to manufacture a) a 5% and b) a 2% dusting agent:

a) 5 parts of the active ingredient of Formula I
95 parts of talc;

b) 2 parts of the active ingredient
1 part of highly dispersed silica
97 parts of talc.

The active ingredients are mixed and ground with the carriers and can in this form be scattered as dust as necessary for the application.

*Granulate:*

The following materials are used to manufacture a 5% granulate:

5 parts of the active ingredient of Formula I
0.25 parts of epoxylated vegetable oil
0.25 parts of cetyl polyglycol ether
3.50 parts of polyethylene glycol
91 parts of kaolin (particle size 0.3—0.8 mm).

The active substance is mixed with epoxylated vegetable oil and dissolved in 6 parts of acetone. The polyethylene glycol and cetyl polyglycol ether are added next. The resulting solution is sprayed onto kaolin and the acetone is evaporated off under vacuum. A microgranulate of this kind is used advantageously for combatting soil fungi.

*Wettable powder:*

The following components are used to manufactuer a) a 70%, b) a 40%, c) and d) a 25%, and e) a 10% wettable powder:

70 parts of the active ingredient of Formula I
5 parts of sodium dibutylnaphthylsulfonate
3 parts of naphthalenesulfonic acids-phenolsulfonic acids-formaldehyde condensate (3:2:1)
10 parts of kaolin
12 parts of Champagne chalk;

b) 40 parts of the active ingredient of Formula I
5 parts of sodium ligninsulfonate
1 part of sodium dibutylnaphthalenesulfonate
54 parts of silica;

c) 23 parts of the active ingredient of Formula I
4.5 parts of calcium ligninsulfonate
1.9 parts of Champagne chalk/hydroxyethylcellulose mixture (1:1)
1.5 parts of sodium dibutylnaphthalenesulfonate
19.5 parts of silica
19.5 parts of Champagne chalk
28.1 parts of kaolin;

d) 25 parts of the active ingredient of Formula I
2.5 parts of isooctylphenoxypolyoxyethyleneethanol
1.7 parts of Champagne chalk/hydroxyethylcellulose mixture (1:1)
8.3 parts of sodium aluminium silicate
16.5 parts of kieselguhr
46 parts of kaolin;

e) 10 parts of the active ingredient of Formula I
3 parts of a mixture of sodium salts of saturated fatty alcohol sulfates
5 parts of naphthalenesulfonic acid/formaldehyde condensate
82 parts of kaolin.

The active ingredients are mixed thoroughly with the additives in suitable mixers and ground in appropriate mills and rollers. Wettable powders of excellent wettability and buoyancy are obtained,

which can be diluted with water to suspensions of the desired concentration and are particularly suitable for foliar application.

*Emulsifiable concentrates:*

The following materials are used to manufacture a 25% emulsifiable concentrate:

25 parts of active ingredient of Formula I
2.5 parts of epoxydized vegetable oil
10 parts of an alkylarylsulfonate/fatty alcohol polyglycol ether mixture
5 parts of dimethylformamide
57.5 parts of xylene

Emulsions of the concentration desired for use can be prepared from concentrates of this type by dilution with water. These are particularly suited for foliar application

## Biological Examples

### Example 2

*Action against Erysiphe graminis on barley*

a) Residual-protective action

Barley plants about 8 cm high were sprayed with a solution prepared from wettable powder of the active ingredient (0.02% of the active substance). After 4 h the treated plants were dusted with conidia of the fungus. The infected barley was kept in a greenhouse at about 22°C and after 10 days the fungal infection was evaluated.

b) Systemic action

A spray solution made from wettable powder of the active ingredient (0.006% of the active substance referred to the soil volume) was poured over barley plants some 8 cm high. Care was taken that the spray solution did not come into contact with the parts of the plant above the ground. After 48 h the treated plants were dusted with conidia of the fungus. The infected barley was kept in a greenhouse at about 22°C and the fungal infection was evaluated after 10 days.

### Example 3

*Action against Cercospora arachidicola on peanut plants*

a) Residual-protective action

Peanut plants 10—15 cm high were sprayed with a solution (0.02% of the active substance) made from wettable powder of the active substance and infected with a fungal conidia suspension 48 h later. The infected plants were incubated for 72 h at about 21°C and a high relative humidity and were kept in a greenhouse until the appearance of the typical spots on the leaves. The fungicidal action was evaluated 12 days after the infection, on the basis of the number and size of the spots.

### Example 4

*Action against Puccinia graminis on wheat*

Wheat plants were sprayed with a solution made from wettable powder of the active ingredient (0.06% of the active substance) 6 days after sowing. After 24 h the treated plants were infected with a urediospore suspension of the fungus. After incubation for 48 h at 95—100% relative humidity and about 20°C the infected plants were kept in a greenhouse at about 22°C.

The evaluation of the rust pustule development took place 12 days after the infection.

b) Systemic action

5 days after sowing a solution prepared from the wettable powder of the active ingredient (0.006% of the active substance referred to the soil volume) was poured over wheat plants. After 48 h the treated plants were infected with a urediospore suspension of the fungus. After incubation for 48 h at 95—100% relative humidity and about 20°C, the infected plants were placed in a greenhouse at about 22°C. The evaluation of the rust pustule development took place 12 days after the infection.

### Example 5

*Action on Botrytis cinerea on broad beans Residual-protective action*

Broad bean plants about 10 cm in height were sprayed with a solution prepared from the wettable powder of the active substance (0.02% of the active substance). After 24 h the treated plants were infected with a fungal conidia suspension. After incubating the infected plants for 2—3 days at 95—100% relative humidity and 21°C, the fungal infection was evaluated.

### Example 6

*Action of Venturia inaequalis on apple trees Residual-protective action*

Apple seedlings with about 5 developed leaves were sprayed with a solution prepared from

wettable powder of the active substance (0.006%, 60 ppm) of the active substance. After 24 h the treated plants were infected with a conidia suspension of the fungus. The plants were then incubated at 90—100% relative humidity for 5 days and kept in a greenhouse at 20—24°C for a further 10 days. The scab formation was evaluated 15 days after the infection.

According to the discovery, the prior experiments show in general a good fungicide activity. Among others the below listed compounds achieved a retardation of the attack of less than 20% in comparison with untreated but infected control plants. By the compounds marked with asterisk no attack occurred.

By Erysiphe graminis: No. 1.2*, 1.7*, 1.8, 1.9*, 1.10*, 1.12*, 1.14, 1.15*, 1.16*, 1.17*, 1.18*, 1.20*, 1.21, 1.22, 1.23, 1.24, 1.25, 1.26, 1.27, 1.28, 1.30, 1.31, 1.32, 1.34, 1.35, 1.36, 1.37, 1.38, 1.39*, 1.40, 1.41, 1.43, 1.45, 1.46*, 1.47*, 1.48*, 1.49*, 1.50, 1.51, 1.52, 1.53, 1.57*, 1.58*, 1.60*, 1.69*, 1.80*, 1.81*, 1.82*, 1.84*, 1.85*, 1.86*, 1.88*, 1.92*, 1.94*, 1.97*, 2.1*, 2.10*, 2.11*, 2.12*, 3.7*, 4.1, 4.7*, 4.14*, 4.17*, 4.23*.

By Cercospora arachidicola: No. 1.7, 1.8, 1.14, 1.16*, 1.17*, 1.45, 1.46*, 1.47*, 1.48*, 1.49*, 1.50, 1.51, 1.52, 1.2* 1.9*, 1.10*, 1.12*, 1.15*, 1.18*, 1.20*, 1.39* 1.57*, 1.58*, 1.60*, 1.69* 1.80*, 1.81*, 1.82*, 1.84*, 1.85*, 1.86*, 1.88*, 1.97*, 2.10*, 2.11*, 2.12*, 4.7*, 4.23.

By Puccinia graminis: No. 1.2*, 1.7*, 1.8, 1.14, 1.16*, 1.17*, 1.18*, 1.19, 1.20*, 1.22, 1.23, 1.36, 1.39, 1.41, 1.43, 1.45, 1.46*, 1.47*, 1.48*, 1.49*, 1.9*, 1.10*, 1.12*, 1.15*, 1.57*, 1.85*, 1.60*, 1.69*, 1.80*, 1.81*, 1.84*, 1.85*, 1.86*, 1.88*, 1.92*, 1.93*, 1.94, 1.96, 1.97*, 2.10*, 2.11*, 2.12*, 4.7*, 4.14*, 4.23*.

By Botrytis cinerea: No. 1.14, 1.15, 1.34, 1.36, 1.48*, 1.49*,

By Venturia inaequalis: No. 1.9*, 1.10*, 1.12*, 1.14*, 1.15*, 1.17*, 1.18*, 1.20*, 1.22*, 1.23*, 1.47*, 1.48*, 1.49*, 1.57*, 1.58*, 1.69*, 1.80*, 1.81*, 1.85*, 1.97*, 2.11*, 2.12*, 4.23*.

## Claims

1. Compound of the formula I

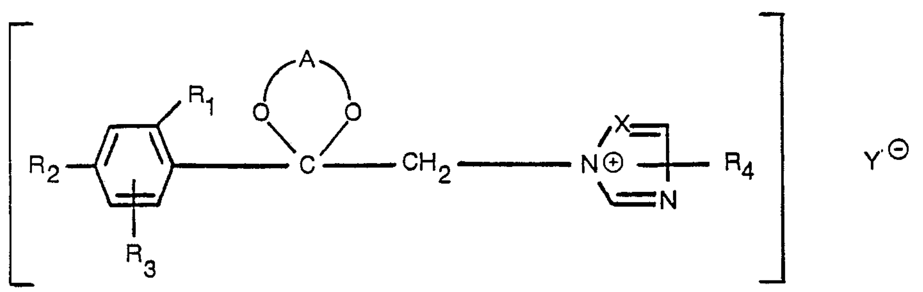

(I)

wherein

$R_1$, $R_2$ and $R_3$ represent independently of one another hydrogen or halogen, at least one residue being a halogen,

A represents one of the following groups a), b), c) and d)

(a)

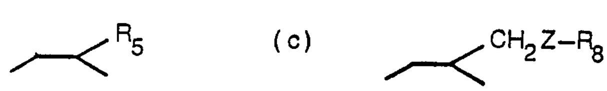

(c)

(b)

(d)

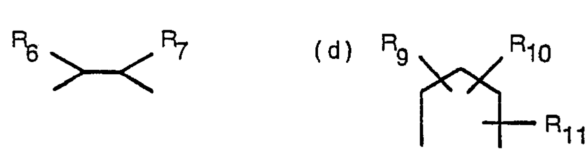

wherein

Z represents O or S,

$R_5$ represents hydrogen or $C_1$—$C_6$-alkyl, possibly substituted with a halogen;

$R_6$ represents methyl or ethyl,

$R_7$ represents methyl, ethyl or propyl, or

$R_6$ and $R_7$ together form a tetramethylene group which can be substituted by 1 or 2 methyl groups,

$R_8$ represents $C_1$—$C_6$-alkyl, $C_3$—$C_4$-alkenyl, 2-propynyl, 3-halo-2-propynyl, $C_1$—$C_6$-alkoxy-$C_1$—$C_6$-alkyl or phenyl or benzyl possibly substituted singly, doubly, or triply by halogen, alkyl, alkoxy, nitro, cyano or trifluoromethyl,

$R_9$ and $R_{10}$ represent independently of one another hydrogen or $C_1$—$C_4$-alkyl and

$R_{11}$ represents hydrogen, $C_1$—$C_4$-alkyl or —$CH_2OH$, the total number of C atoms in $R_9$, $R_{10}$ and $R_{11}$ not exceeding 10,

X represents —CH= or —N=,

$R_4$ represents $C_1$—$C_{16}$-alkyl, possibly substituted singly or multiply by halogen, CN, SCN, COO($C_1$—$C_3$)-alkyl, phenyl or naphthyl, and/or possibly interrupted by O, S,

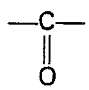 or

$SO_2$, $C_3$—$C_6$-alkenylmethyl possibly substituted by a halogen or an alkylthio group, or $C_3$—$C_6$-alkynylmethyl possibly substituted by a halogen, the phenyl or naphthyl group substituent possible in $R_4$ being also possibly substituted singly or multiply by halogen, $NO_2$, CN, OH, $C_1$—$C_4$-alkyl, methoxy, trifluoromethyl or phenyl, and

Y represents the anion of an inorganic or organic acid.

2. A compound of the formula I according to claim 1, wherein

$R_1 = R_2 =$ chlorine,

$R_3 =$ hydrogen,

A = a) 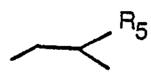

b) 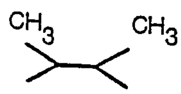 or 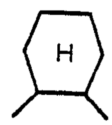

c) 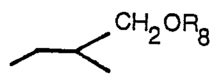

d) 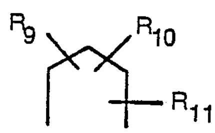

wherein

$R_5$ represents hydrogen or $C_1$—$C_4$-alkyl,

$R_8$ represents $C_1$—$C_3$-alkyl or phenyl,

$R_9$ and $R_{10}$ independently of one another represent hydrogen or $C_1$—$C_4$-alkyl and

$R_{11}$ represents hydrogen, $C_1$—$C_4$-alkyl or $CH_2OH$, with the proviso that the total number of C atoms in $R_9$, $R_{10}$ and $R_{11}$ does not exceed 10,

X represents —N=,

$R_4$ represents $C_3$—$C_6$-alkenylmethyl, $C_3$—$C_6$-alkynylmethyl,

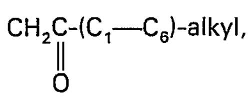, 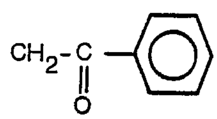

(optionally substituted by Cl, $NO_2$, CN, OH, $CH_3$ or $OCH_3$) or $CH_2CN$ and

Y represents a halide, sulphate, nitrate, phosphate, methanesulphonate, ethanesulphonate, p-toluenesulphonate or benzenesulphonate anion.

3. A compound of the formula I according to Claim 1, wherein

$R_1 =$ hydrogen or chlorine,

$R_2 =$ chlorine,

$R_3 =$ hydrogen,

A = e) 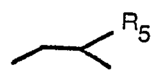

f) 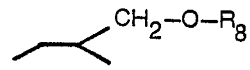

g) 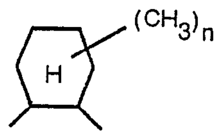 n = 0 or 1

h) 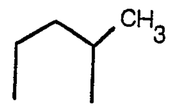

i) 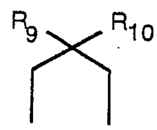

wherein

$R_5$ represents ethyl or n-propyl,

$R_8$ $C_1$—$C_3$-alkyl or phenyl,

$R_9$ represents hydrogen or methyl and

$R_{10}$ represents hydrogen or $C_1$—$C_3$-alkyl,

X = N and

$R_4$ = —$CH_2$—CO—$R_{12}$ or —CH($COOR_{13}$)

wherein

$R_{13}$ is $C_1$—$C_4$-alkyl or a phenyl radical which is unsubstituted or is monosubstituted or polysubstituted by alkyl, halogen, $NO_2$, CN, OH, $OCH_3$ or $CF_3$,

$R_{12}$ furthermore represents —$CH_2$—CO—$R_{13}$, —$CH_2$—$COOR_{13}$ or —$OR_{14}$,

$R_{13}$ represents $C_1$—$C_4$-alkyl and

$R_{14}$ represents alkyl, alkenyl or alkynyl, each of at most 6 C atoms, or an unsubstituted or $C_1$—$C_4$-alkyl- and/or halogen-substituted phenyl radical.

4. A compound according to claim 1 from the group

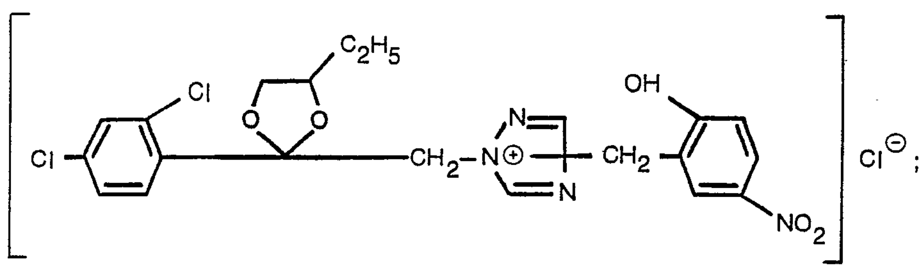

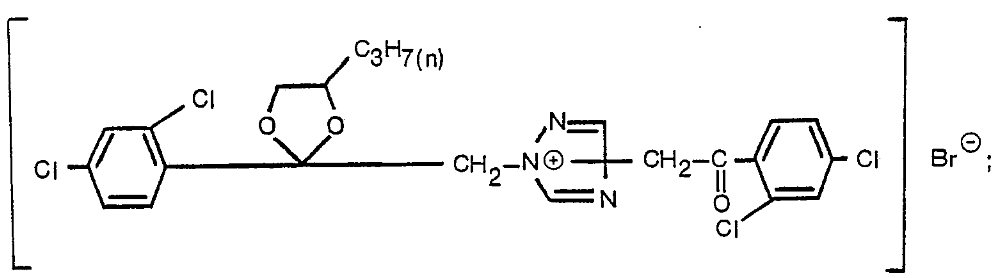

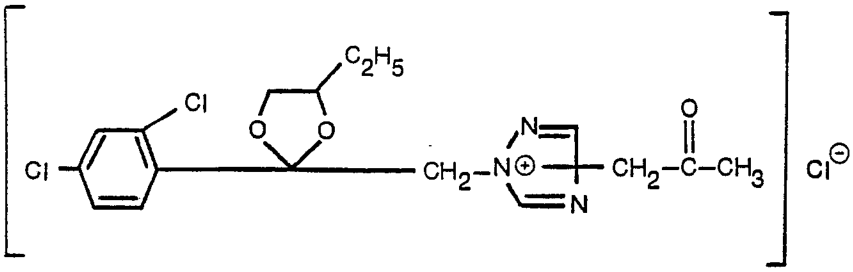
;

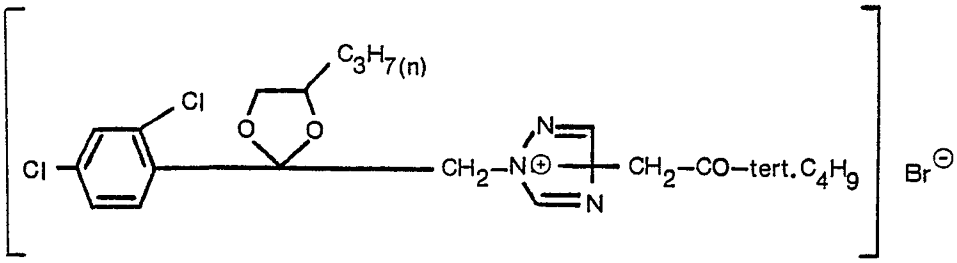
;

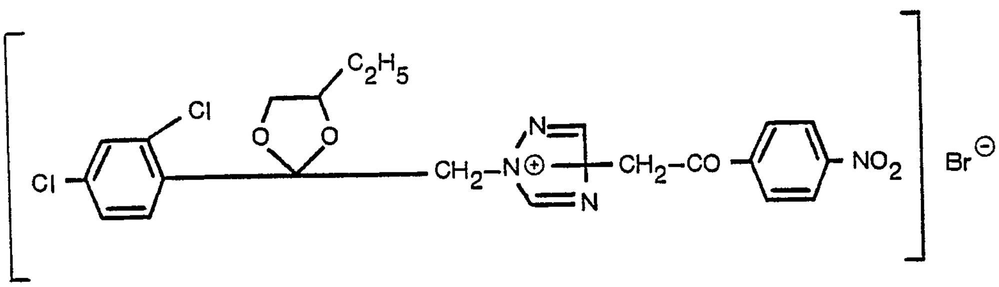
;

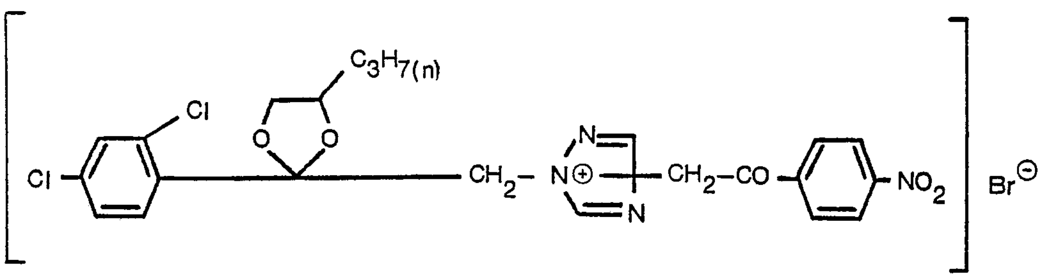
;

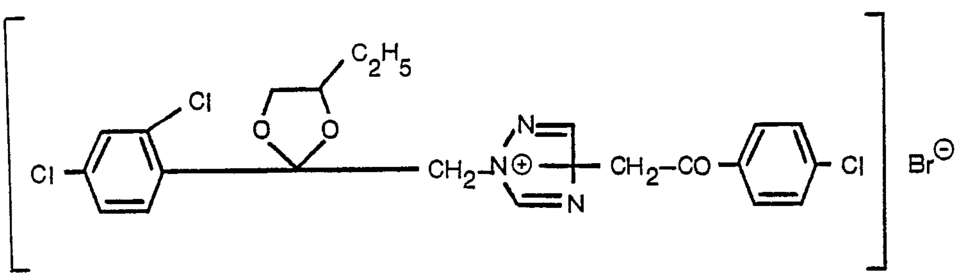
;

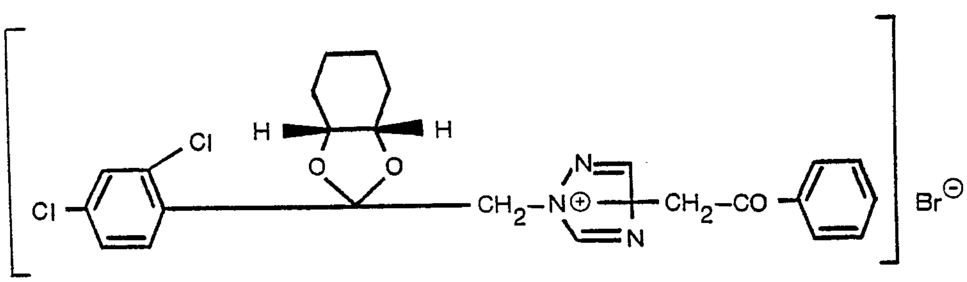
;

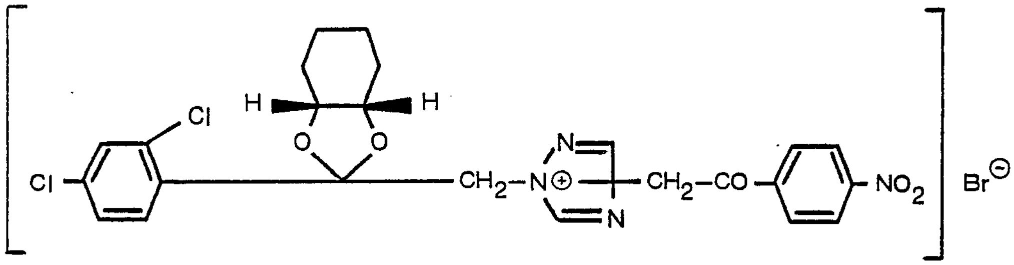
;

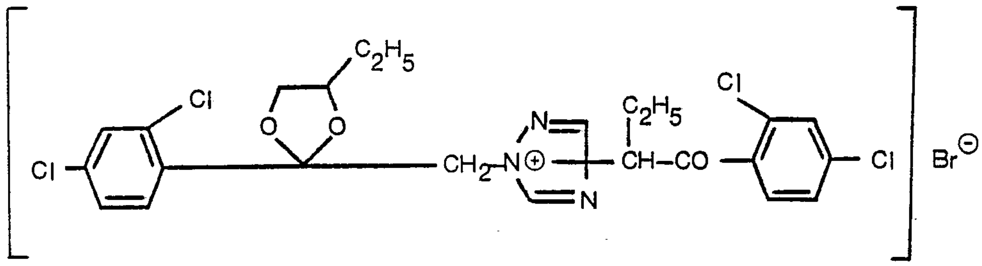
;

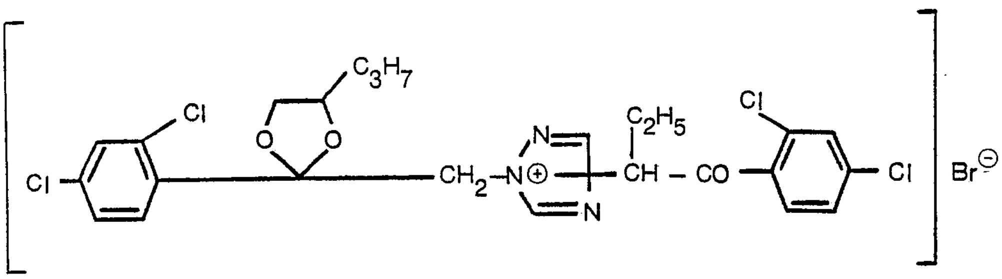
;

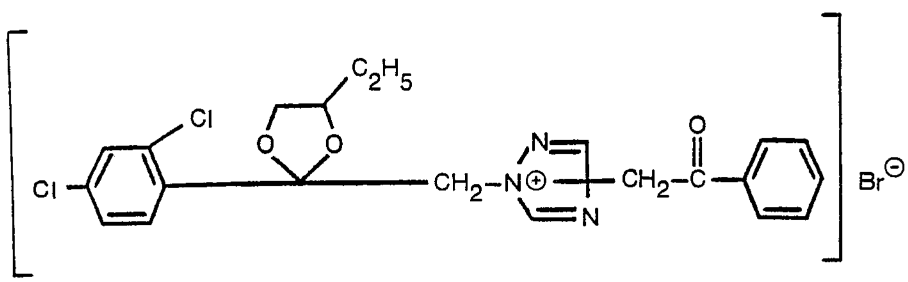
;

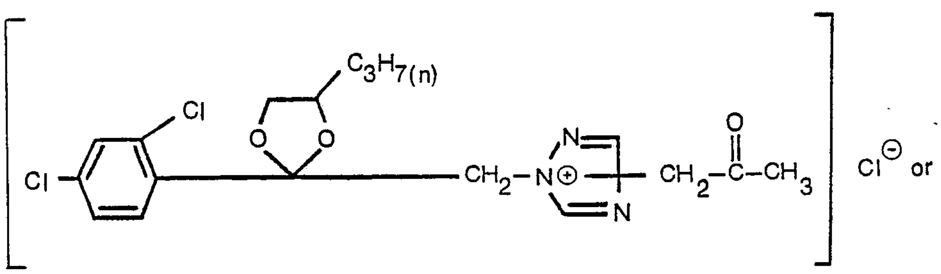
or

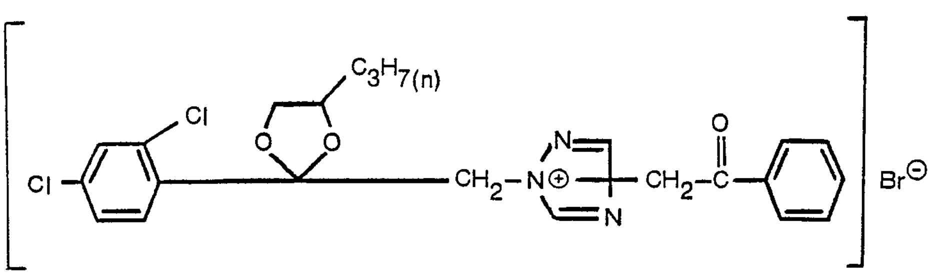

5. Process for the preparation of compounds of the formula I, characterised in that a compound of the formula II

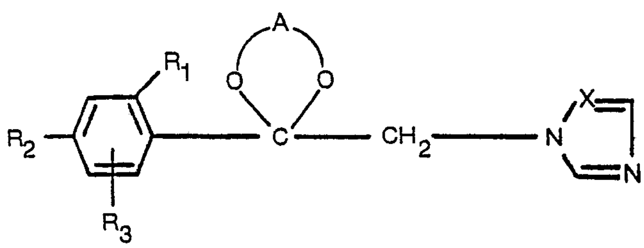

(II)

is reacted with a compound of the formula III

$$Y—R_4 \quad \text{(III)}$$

wherein

$R_1$, $R_2$, $R_3$, $R_4$, A, X and Y have the meanings given under formula I.

6. Agent for combating and/or preventing attack by phytopathogenic fungi, characterised in that it contains, as at least one active component, a compound of the formula I according to one of Claims 1 to 4.

7. Agent according to Claim 6, characterised in that it contains 0.1 to 95 per cent by weight of an active compound of the formula I according to one of Claims 1 to 4, 5 to 99.9 per cent by weight of additives wherein the additives include 0 to 30 per cent by weight of a tenside.

8. Process for the preparation of agents for combating and/or preventing attack by phytopathogenic fungi, characterised in that the active compound of the formula I according to one of Claims 1 to 4 is intimately mixed with carriers and/or tensides.

9. Use of a compound of the formula I according to one of Claims 1 to 4 for combating phytopathogenic fungi.

10. Process for combating and/or preventing attack of the plants by phytopathogenic fungi, characterised in that a compound of the formula I according to one of Claims 1 to 4 is applied, in a diluted form, to the plant or it its locus.

**Revendications**

1. Composé de formule I

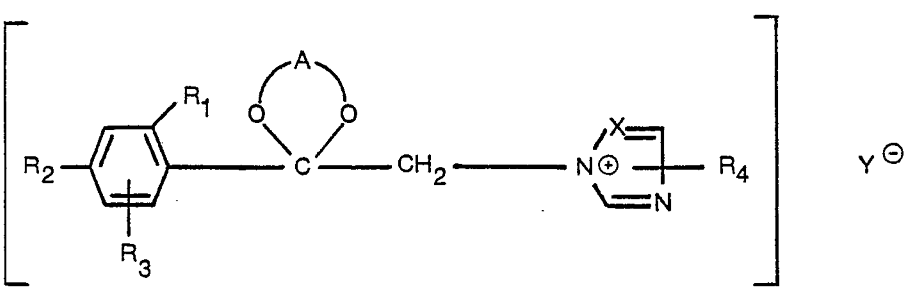

(I)

dans laquelle $R_1$, $R_2$ et $R_3$ représentent indépendamment les uns des autres un hydrogène ou un halogène, au moins un reste étant un halogène;

A représente un des groupes (a), (b), (c) et (d) suivants:

(a) 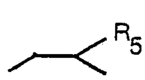 (c) 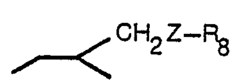

(b) 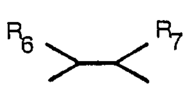 (d) 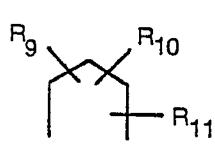

où

Z représente O ou S;

$R_5$ représente un hydrogène ou un alkyle en $C_1$—$C_6$, éventuellement substitué par un halogène;

$R_6$ représente un méthyle ou un éthyle;

$R_7$ représente un méthyle, un éthyle ou un propyle, ou $R_6$ et $R_7$ forment ensemble un groupe tétraméthylène qui peut être substitué par 1 ou 2 groupes méthyles;

$R_8$ représente un alkyle en $C_1$—$C_6$, un alcényle en $C_3$—$C_4$, un 2-propynyle, un 3-halogéno-2-propynyle, un alcoxy($C_1$—$C_6$) alkyl($C_1$—$C_6$), ou un phényle ou benzyle éventuellement substitué 1, 2 ou 3 fois par un halogène, un alkyle, un alcoxy, un nitro, un cyano ou un trifluorométhyle;

$R_9$ et $R_{10}$ représentent indépendamment l'un de l'autre un hydrogène ou un alkyle en $C_1$—$C_4$, et

$R_{11}$ représente un hydrogène, un alkyle en $C_1$—$C_4$ ou —$CH_2OH$, le nombre total des atomes de carbone dans $R_9$, $R_{10}$ et $R_{11}$ ne dépassant pas 10;

X représente —CH= ou —N=;

$R_4$ représente un alkyle en $C_1$—$C_{16}$ éventuellement substitué une ou plusieurs fois par un halogène, un CN, un SCN, un COO alkyle (en $C_1$—$C_3$), un phényle ou un naphtyle et/ou éventuellement interrompu par O, S,

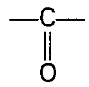 ou $SO_2$,

un alcénylméthyle (en $C_3$—$C_6$) éventuellement substitué par un halogène ou un groupe alkylthio, ou un alcynylméthyle (en $C_3$—$C_6$), éventuellement substitué par un halogène, le groupe phényle ou naphtyle substituant éventuellement $R_4$ étant également éventuellement substitué une ou plusieurs fois par un halogène, un $NO_2$, un CN, un OH, un alkyle en $C_1$—$C_4$, un méthoxy, un trifluorométhyle ou un phényle; et

Y représente l'anion d'un acide organique ou minéral.

2. Un composé de formule I selon la revendication 1, où

$R_1 = R_2 =$ chlore

$R_3 =$ hydrogène

A = a) 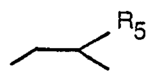

b) 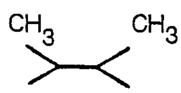 ou 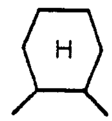

c) 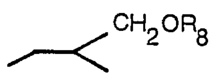

d) 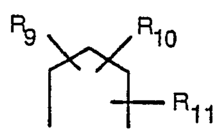

où

$R_5$ représente un hydrogène ou un alkyle en $C_1$—$C_4$,

$R_8$ représente un alkyle en $C_1$—$C_3$ ou un phényle,

$R_9$ et $R_{10}$, indépendamment l'un de l'autre, représentent un hydrogène ou un alkyle en $C_1$—$C_4$ et

$R_{11}$ représente un hydrogène, un alkyle en $C_1$—$C_4$ ou $CH_2OH$, sous réserve que le nombre total des atomes de carbone de $R_9$, $R_{10}$ et $R_{11}$ ne dépasse pas 10,

X représente —N=;

$R_4$ représente un alcénylméthyle (en $C_3$—$C_6$), un alcynylméthyle (en $C_3$—$C_6$), un

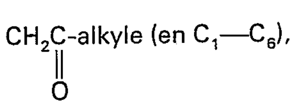, 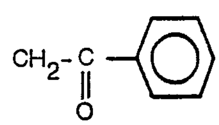

éventuellement substitué par Cl, $NO_2$, CN, OH, $CH_3$ ou $OCH_3$) ou $CH_2CN$, et

Y représente un anion halogénure, sulfate, nitrate, phosphate, méthanesulfonate, éthanesulfonate, p-toluènesulfonate ou benzènesulfonate.

3. Un composé de formule I selon la revendication 1, où

$R_1 =$ un hydrogène ou un chlore,

$R_2$ = un chlore
$R_3$ = un hydrogène,

A = e) 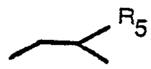

f) 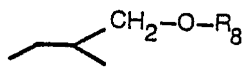

g) 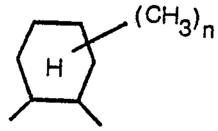 n = 0 ou 1

h) 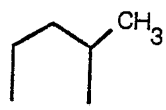

i) 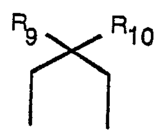

où

$R_5$ représente un éthyle ou un n-propyle,
$R_8$ représente un alkyle en $C_1$—$C_3$ ou un phényle,
$R_9$ représente un hydrogène ou un méthyle, et
$R_{10}$ représente un hydrogène ou un alkyle en $C_1$—$C_3$,
X = N, et
$R_4$ = —$CH_2$—CO—$R_{12}$ ou —CH($COOR_{13}$)

où

$R_{13}$ est un alkyle en $C_1$—$C_4$ ou un radical phényle qui est non substitué ou qui est monosubstitué ou polysubstitué par un alkyle, un halogène, $NO_2$, CN, OH, $OCH_3$ ou $CF_3$,
$R_{12}$ représente de plus —$CH_2$—CO—$R_{13}$, —$CH_2$—$COOR_{13}$ ou —$OR_{14}$,
$R_{13}$ représente un alkyle en $C_1$—$C_4$, et
$R_{14}$ représente un alkyle, un alcényle ou un alcynyle, ayant chacun au plus 6 atomes de carbone, ou un radical phényle non substitué ou substitué par un alkyle en $C_1$—$C_4$ et/ou un halogène.

4. Un composé selon la revendication 1, du groupe

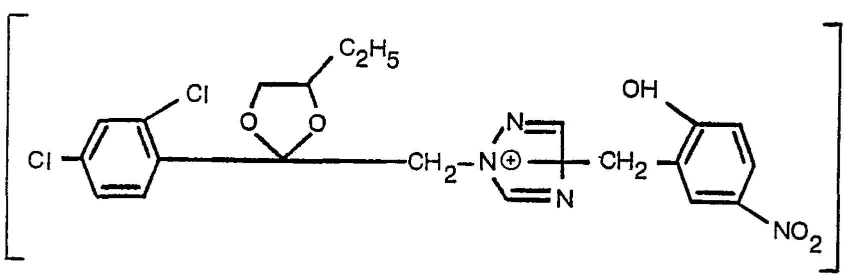 $Cl^{\ominus}$ ;

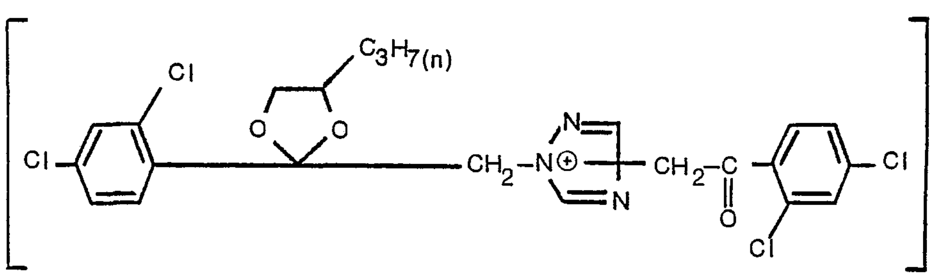 $Br^{\ominus}$ ;

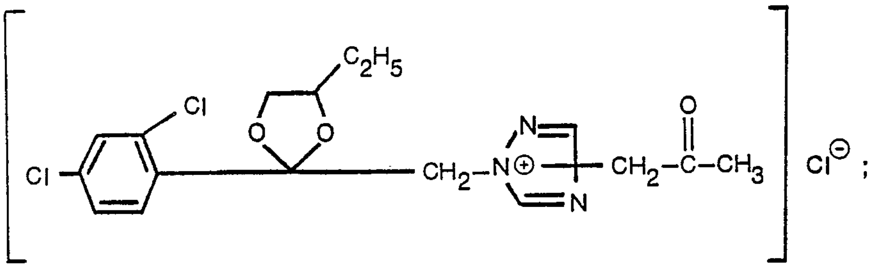

h)

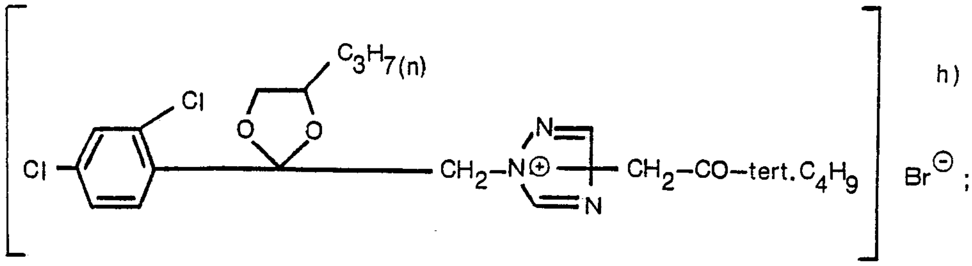

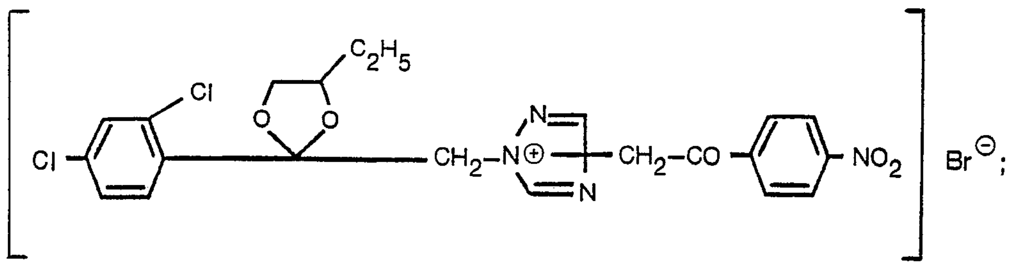

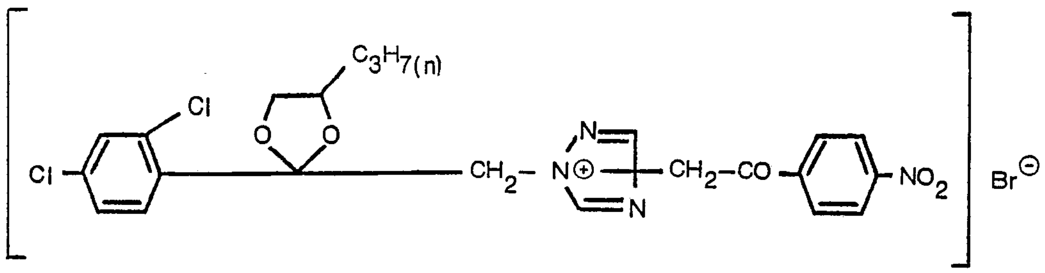

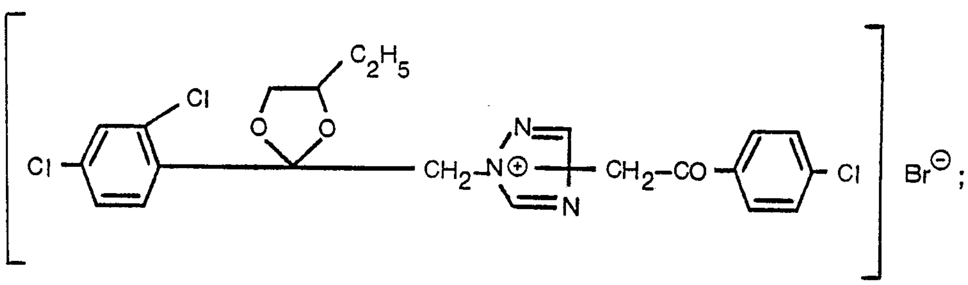

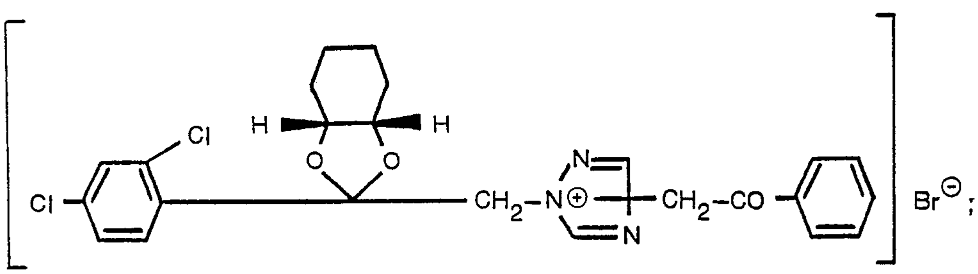

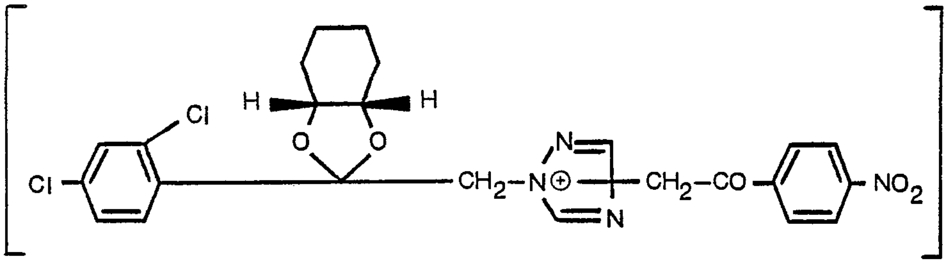

$Br^{\ominus}$ ;

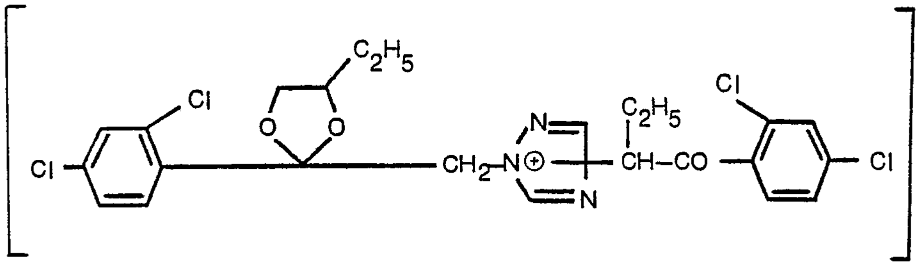

$Br^{\ominus}$ ;

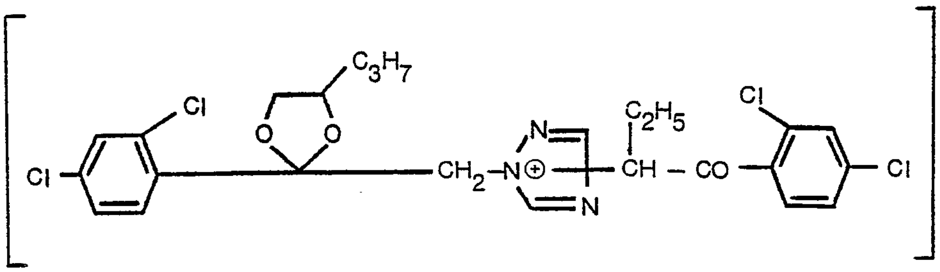

$Br^{\ominus}$ ;

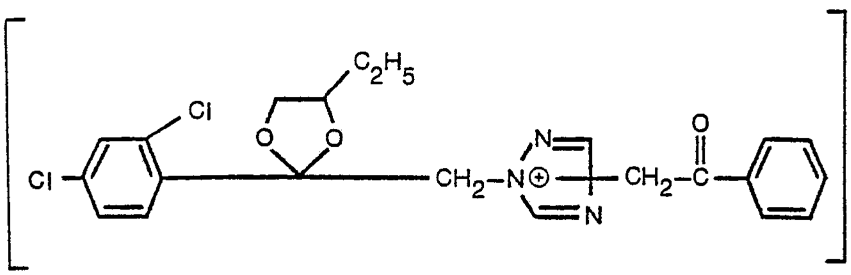

$Br^{\ominus}$ ;

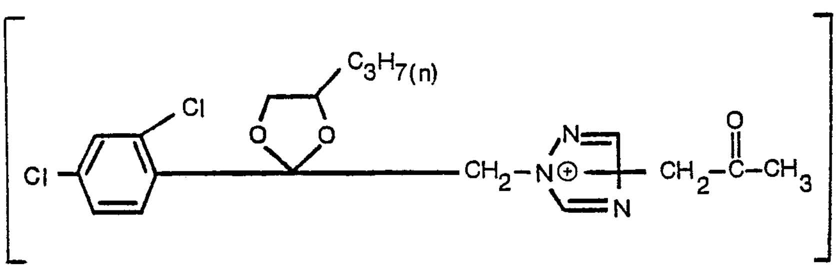

$Cl^{\ominus}$ ou

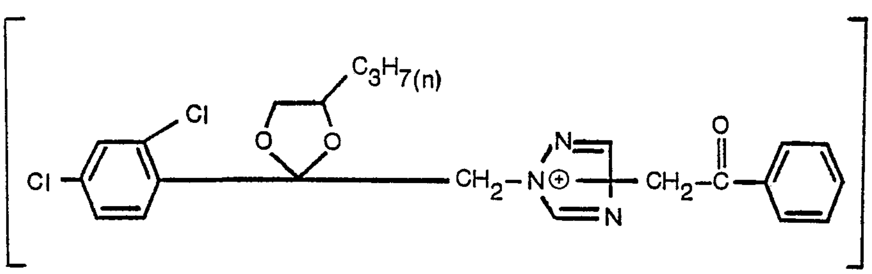

$Br^{\ominus}$

5. Procédé pour la préparation des composés de formule I, caractérisé en ce qu'on fait réagir un composé de formule II

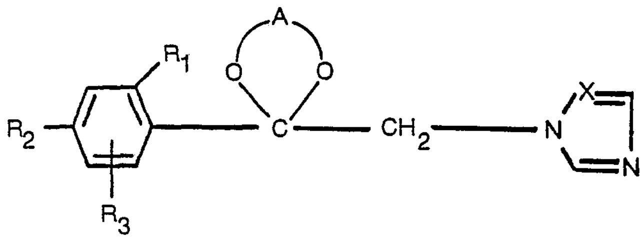

(II)

avec un composé de formule III

$$Y—R_4 \qquad (III)$$

où $R_1$, $R_2$, $R_3$, $R_4$, A, X et Y ont les significations indiquées sous la formule I.

6. Agent pour combattre et/ou éviter l'attaque par les champignons phytopathogènes caractérisé en ce qu'il contient, comme au moins un composant actif, un composé de formule I selon l'une des revendications 1 à 4.

7. Agent selon la revendication 6, caractérisé en ce qu'il contient 0,1 à 95% en poids d'un composé actif de formule I selon l'une des revendications 1 à 4 et 5 à 99,9% en poids d'additifs, les additifs comprenant 0 à 30% en poids d'un tenside.

8. Procédé pour la préparation d'agents pour combattre et/ou éviter l'attaque par les champignons phytopathogènes, caractérisé en ce que le composé actif de formule I selon une des revendications 1 à 4 est mélangé intimement avec des supports et/ou des tensides.

9. Emploi d'un composé de formule I selon l'une des revendications 1 à 4 pour lutter contre les champignons phytopathogènes.

10. Procédé pour combattre et/ou éviter l'attaque des végétaux par les champignons phytopathogènes, caractérisé en ce qu'on applique un composé de formule I selon l'une des revendications 1 à 4, sous une forme diluée, au végétal ou à son site.

**Patentansprüche**

1. Verbindung der Formel (I)

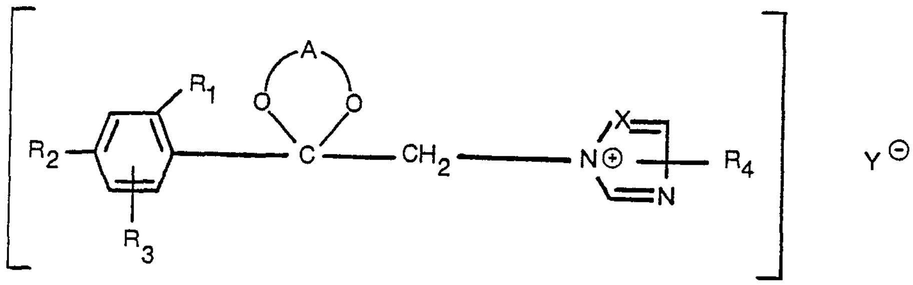

(I)

worin

$R_1$, $R_2$ und $R_3$ unabhängig voneinander Wasserstoff oder Halogen bedeuten, wobei wenigstens ein Rest ein Halogen ist,

A eine der folgenden Gruppen a), b), c) und d) ist:

(a) 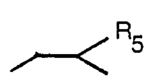 (c) 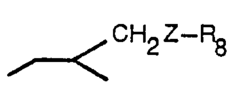

(b) 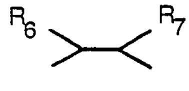 (d) 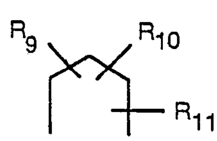

worin

Z für O oder S steht,

$R_5$ Wasserstoff oder $C_1$—$C_6$-Alkyl darstellt, gegebenenfalls substituiert mit einem Halogen;

$R_6$ Methyl oder Ethyl bedeutet,

$R_7$ Methyl, Ethyl oder Propyl darstellt, oder

$R_6$ und $R_7$ gemeinsam eine Tetramethylengruppe bilden, die durch 1 oder 2 Methylgruppen substituiert sein kann,

$R_8$ für $C_1$—$C_6$-Alkyl, $C_3$—$C_4$-Alkenyl, 2-Propinyl, 3-Halogen-2-propinyl, $C_1$—$C_6$-Alkoxy-$C_1$—$C_6$-alkyl oder Phenyl oder Benzyl steht, die gegebenenfalls einfach, zweifach oder dreifach durch Halogen, Alkyl, Alkoxy, Nitro, Cyano oder Trifluormethyl substituiert sind,

$R_9$ und $R_{10}$ unabhängig voneinander Wasserstoff oder $C_1$—$C_4$-Alkyl bedeuten und

$R_{11}$ für Wasserstoff, $C_1$—$C_4$-Alkyl oder —$CH_2OH$ steht, wobei die Gesamtanzahl der Kohlenstoffatome in den Resten $R_9$, $R_{10}$ und $R_{11}$ die Zahl 10 nicht überschreitet,

X für —CH= oder —N= steht,

$R_4$ für $C_1$—$C_{16}$-Alkyl steht, das gegebenenfalls einfach oder mehrfach durch Halogen, CN, SCN, COO($C_1$—$C_3$)-Alkyl, Phenyl oder Naphthyl substituiert und/oder gegebenenfalls durch O, S

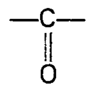 oder $SO_2$

unterbrochen ist, für $C_3$—$C_6$-Alkenylmethyl steht, das gegebenenfalls durch ein Halogen oder eine Alkylthiogruppe substituiert ist, oder für $C_3$—$C_6$-Alkinylmethyl steht, das gegebenenfalls durch ein Halogen substituiert ist, wobei der im Rest $R_4$ mögliche Phenyl- oder Naphthylsubstituent ebenso gegebenenfalls einfach oder mehrfach durch Halogen, $NO_2$, CN, OH, $C_1$—$C_4$-Alkyl, Methoxy, Trifluormethyl oder Phenyl substituiert ist, und

Y das Anion einer anorganischen oder organischen Säure bedeutet.

2. Eine Verbindung der Formel (I) gemäß Anspruch 1, worin

$R_1 = R_2 =$ Chlor darstellt.

$R_3$ für Wasserstoff steht,

A für a) 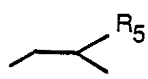

b) 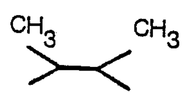 oder 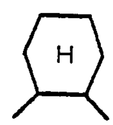

c) 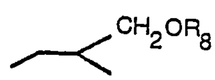

oder d) 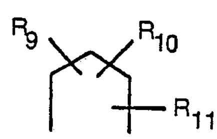

steht, worin

$R_5$ Wasserstoff oder $C_1$—$C_4$-Alkyl darstellt,

$R_8$ $C_1$—$C_3$-Alkyl oder Phenyl bedeutet,

$R_9$ und $R_{10}$ unabhängig voneinander Wasserstoff oder $C_1$—$C_4$-Alkyl bedeuten und

$R_{11}$ für Wasserstoff, $C_1$—$C_4$-Alkyl oder $CH_2OH$ steht, mit der Maßgabe, daß die Gesamtanzahl der Kohlenstoffatome in den Resten $R_9$, $R_{10}$ und $R_{11}$ die Zahl 10 nicht überschreitet,

X für —N= steht,

$R_4$ für $C_3$—$C_6$-Alkenylmethyl, $C_3$—$C_6$-Alkinylmethyl,

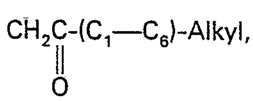, 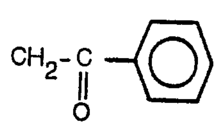

(gegebenenfalls substituiert durch Cl, $NO_2$, CN, OH, $CH_3$ oder $OCH_3$) oder für $CH_2CN$ steht und

Y ein Halogenid-, Sulfat-, Nitrat-, Phosphat-, Methansulfonat-, Ethansulfonat-, p-Toluolsulfonat- oder Benzolsulfonatanion bedeutet.

3. Eine Verbindung der Formel (I) gemäß Anspruch 1, worin
$R_1$ für Wasserstoff oder Chlor steht,
$R_2$ für Chlor steht,
$R_3$ für Wasserstoff steht,

A für e) 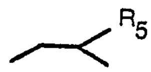

f) 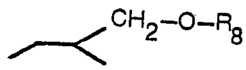

g) 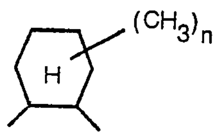 n = 0 oder

h) 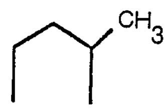

oder

i) 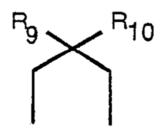

steht, worin
$R_5$ Ethyl oder n-Propyl darstellt,
$R_8$ $C_1$—$C_3$-Alkyl oder Phenyl bedeutet,
$R_9$ Wasserstoff oder Methyl bedeutet und
$R_{10}$ Wasserstoff oder $C_1$—$C_3$-Alkyl darstellt,
X für N steht und
$R_4$ für —$CH_2$—CO—$R_{12}$ oder —CH($COOR_{13}$) steht,
worin
$R_{13}$ für $C_1$—$C_4$-Alkyl oder einen Phenylrest steht, der unsubstituiert oder einfach oder mehrfach durch Alkyl, Halogen, $NO_2$, CN, OH, $OCH_3$ oder $CF_3$ substituiert ist,
$R_{12}$ überdies für —$CH_2$—CO—$R_{13}$, —$CH_2$—$COOR_{13}$ oder —$OR_{14}$ steht,
$R_{13}$ $C_1$—$C_4$-Alkyl darstellt und
$R_{14}$ Alkyl, Alkenyl oder Alkinyl mit jeweils höchstens 6 Kohlenstoffatomen oder einen unsubstituierten oder durch $C_1$—$C_4$-Alkyl und/oder Halogen substituierten Phenylrest darstellt.

4. Eine Verbindung gemäß Anspruch 1 aus der Gruppe

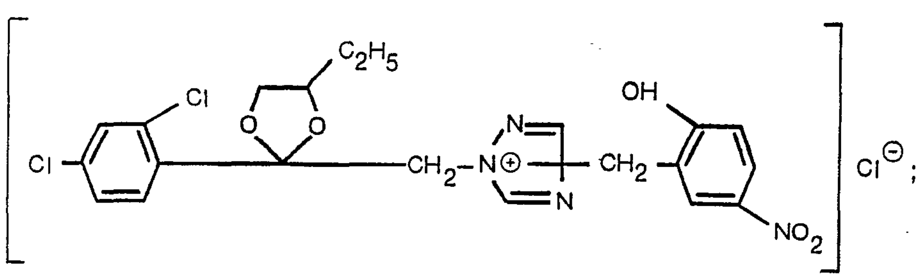

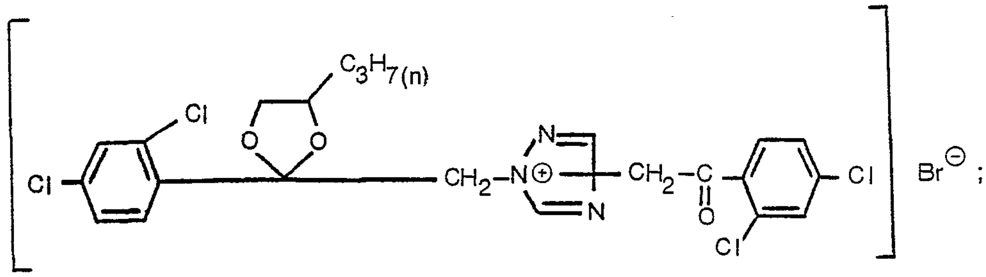

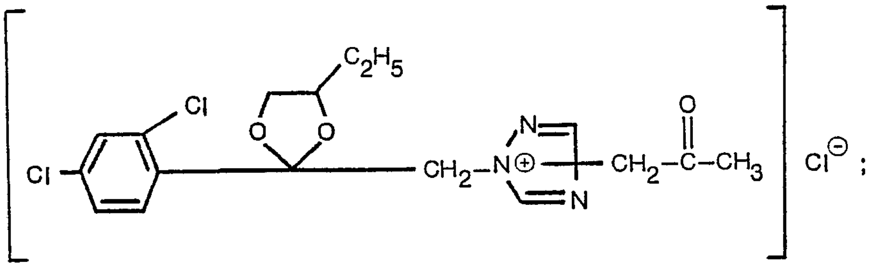

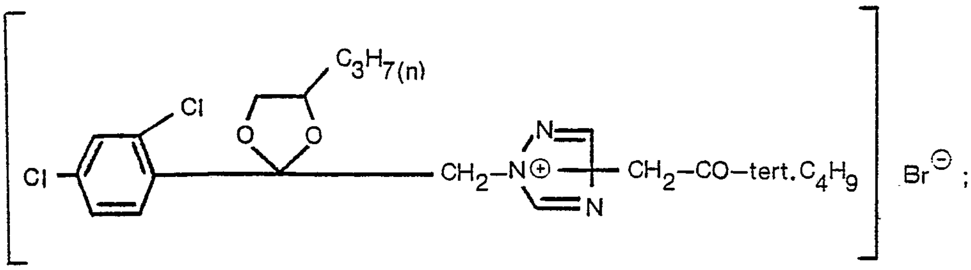

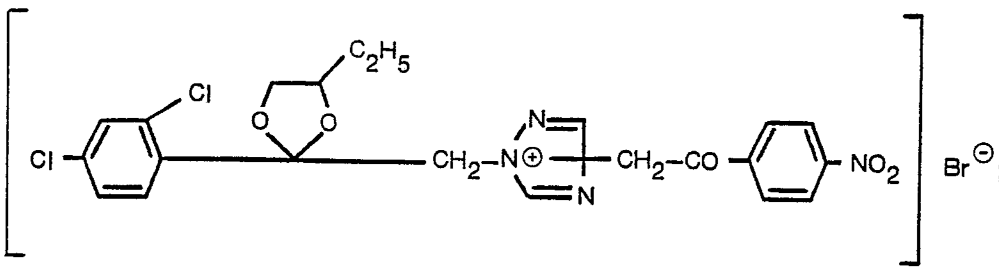

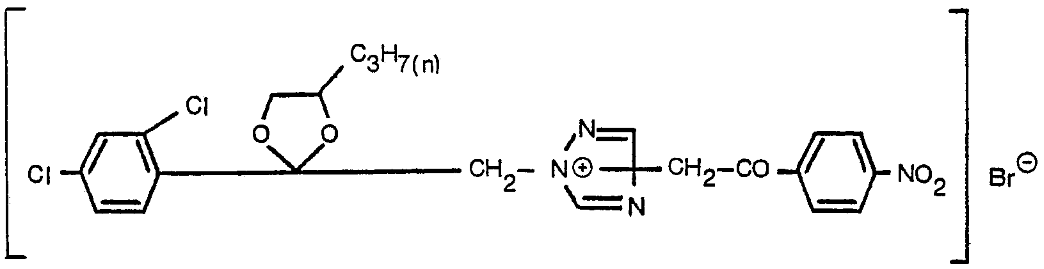

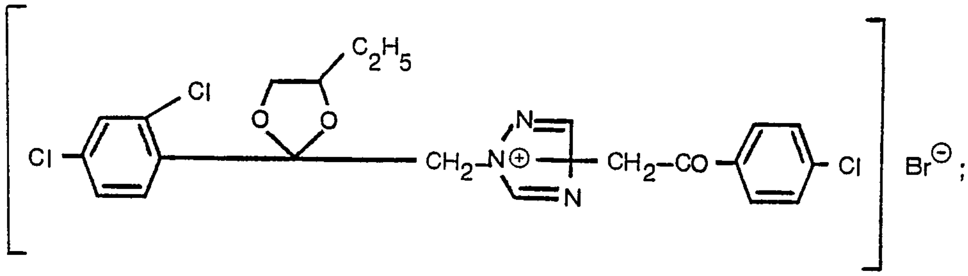

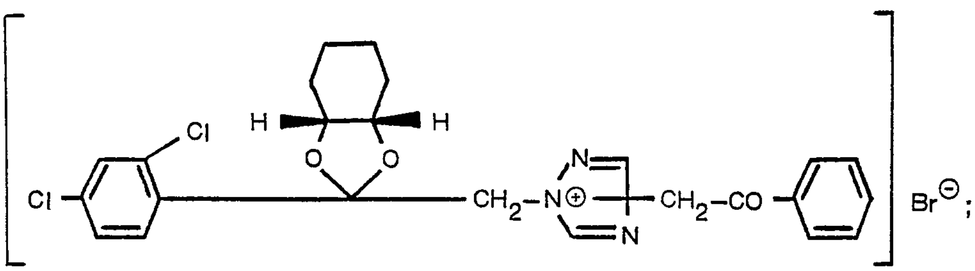

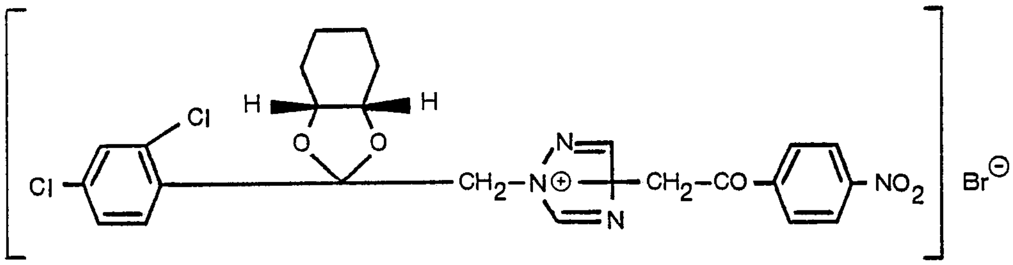

;

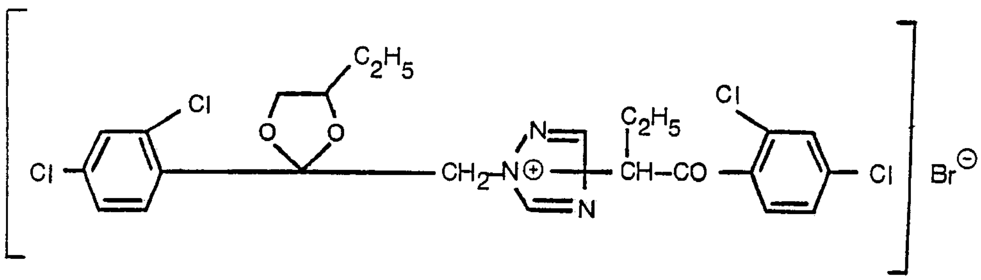

;

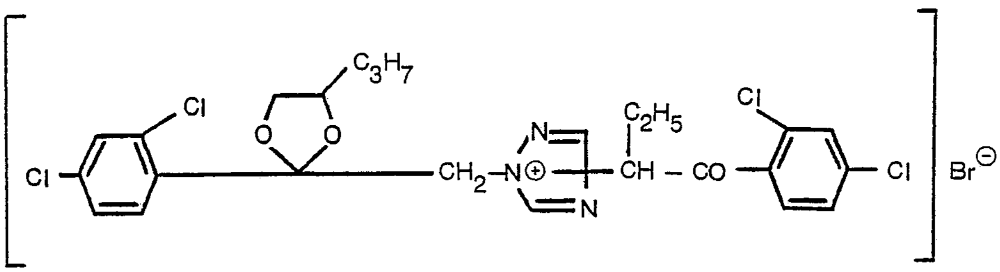

;

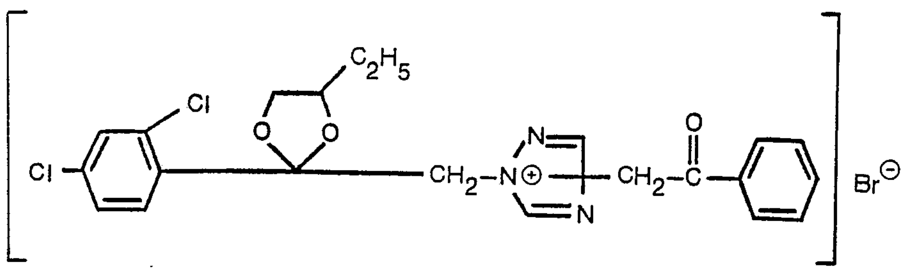

;

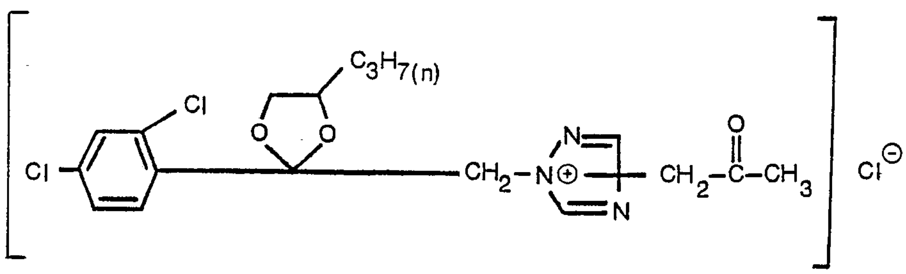

oder

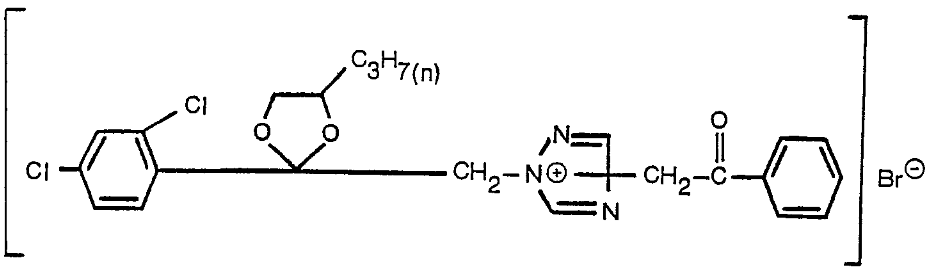

5. Verfahren zur Herstellung von Verbindungen der Formel (I), dadurch gekennzeichnet, daß eine Verbindung der Formel (II)

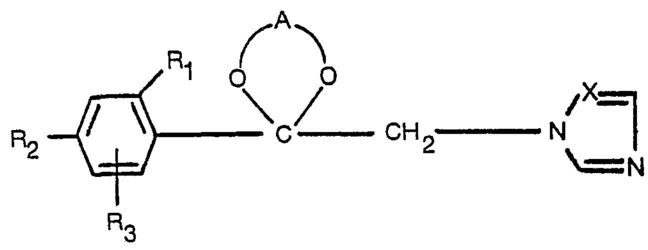

(II)

mit einer Verbindung der Formel (III)

$$Y—R_4 \qquad (III)$$

worin $R_1$, $R_2$, $R_3$, $R_4$, A, X und Y die unter Formel (I) angegebenen Bedeutungen besitzen, umgesetzt wird.

6. Mittel zur Bekämpfung und/oder Verhinderung eines Angriffes durch phytopathogene Pilze, dadurch gekennzeichnet, daß es als wenigstens eine aktive Komponente eine Verbindung der Formel (I) gemäß einem der Ansprüche 1 bis 4 enthält.

7. Mittel gemäß Anspruch 6, dadurch gekennzeichnet, daß es 0,1 bis 95 Gew.-% einer wirksamen Verbindung der Formel (I) gemäß einem der Ansprüche 1 bis 4 und 5 bis 99,9 Gew.-% von Additiven enthält, wobei die Additive 0 bis 30 Gew.-% eines oberflächenaktiven Mittels einschließen.

8. Verfahren zur Herstellung von Mitteln zur Bekämpfung und/oder Verhütung eines Angriffes durch phytopathogene Pilze, dadurch gekennzeichnet, daß die wirksame Verbindung der Formel (I) gemäß einem der Ansprüche 1 bis 4 innig mit Trägern und/oder oberflächenaktiven Mitteln vermischt wird.

9. Verwendung einer Verbindung der Formel (I) gemäß einem der Ansprüche 1 bis 4 zur Bekämpfung phytopathogener Pilze.

10. Verfahren zur Bekämpfung und/oder Verhütung eines Angriffes von phytopathogenen Pilzen auf Pflanzen, dadurch gekennzeichnet, daß eine Verbindung der Formel (I) gemäß einem der Ansprüche 1 bis 4 in verdünnter Form auf die Pflanze oder auf den Ort ihres Wuchses aufgebracht wird.